# EUROPEAN PATENT SPECIFICATION

(11) **EP 2 968 983 B1**
(45) Date of publication and mention of the grant of the patent: **13.02.2019**
(21) Application number: 14720775.7
(22) Date of filing: 13.03.2014
(51) Int. Cl.: A61N 7/02, A61M 25/00, A61M 25/01

(54) **ULTRASONIC CATHETER FOR RENAL DENERVATION**
KATHETER ZUR RENALEN DENERVIERUNG MITTELS ULTRASCHALL
CATHÉTER POUR LA DÉNERVATION RÉNALE ULTRASONIQUE

(30) Priority: 15.03.2013 US 201361801034 P; 14.05.2013 US 201313894094
(43) Date of publication of application: 20.01.2016
(73) Proprietor: Medtronic Ardian Luxembourg S.à.r.l., 2124 Luxembourg (LU)
(72) Inventor: BALLAKUR, Sowmya, Santa Rosa, California 95403 (US); CLARK, Benjamin, Santa Rosa, California 95403 (US)
(74) Representative: Zimmermann & Partner Patentanwälte mbB
(86) International application number: PCT/US2014/025392
(87) International publication number: WO 2014/151295

(56) References cited:
- WO-A1-00/45706
- WO-A2-2007/134258
- WO-A2-2012/120495

## Description

### TECHNICAL FIELD

The present disclosure relates generally to the field of neuromodulation, and some embodiments relate to neuromodulation for renal denervation. More particularly, some embodiments relate to the application of ultrasonic energy to accomplish renal denervation.

### BACKGROUND

The sympathetic nervous system (SNS) is a primarily involuntary bodily control system typically associated with stress responses. Fibers of the SNS innervate tissue present in almost every organ system of the human body and can affect characteristics such as pupil diameter, gut motility, and urinary output. Such regulation can have adaptive utility in maintaining homeostasis or preparing the body for rapid response to environmental factors. Chronic activation of the SNS, however, is a common maladaptive response that can drive the progression of many disease states. Excessive activation of the renal SNS in particular has been identified experimentally and in humans as a likely contributor to the complex pathophysiology of hypertension, states of volume overload (such as heart failure), and progressive renal disease. For example, radiotracer dilution has demonstrated increased renal norepinephrine ("NE") spillover rates in patients with essential hypertension.

Cardio-renal sympathetic nerve hyperactivity can be particularly pronounced in patients with heart failure. For example, an exaggerated NE overflow from the heart and kidneys of plasma is often found in these patients. Heightened SNS activation commonly characterizes both chronic and end stage renal disease. In patients with end stage renal disease, NE plasma levels above the median have been demonstrated to be predictive of cardiovascular diseases and several causes of death. This is also true for patients suffering from diabetic or contrast nephropathy. Evidence suggests that sensory afferent signals originating from diseased kidneys are major contributors to initiating and sustaining elevated central sympathetic outflow.

Sympathetic nerves innervating the kidneys terminate in the blood vessels, the juxtaglomerular apparatus, and the renal tubules. Stimulation of the renal sympathetic nerves can cause increased renin release, increased sodium (Na⁺) reabsorption, and a reduction of renal blood flow. These neural regulation components of renal function are considerably stimulated in disease states characterized by heightened sympathetic tone and likely contribute to increased blood pressure in hypertensive patients. The reduction of renal blood flow and glomerular filtration rate as a result of renal sympathetic efferent stimulation is likely a cornerstone of the loss of renal function in cardio-renal syndrome (i.e., renal dysfunction as a progressive complication of chronic heart failure). Pharmacologic strategies to thwart the consequences of renal efferent sympathetic stimulation include centrally acting sympatholytic drugs, beta blockers (intended to reduce renin release), angiotensin converting enzyme inhibitors and receptor blockers (intended to block the action of angiotensin II and aldosterone activation consequent to renin release), and diuretics (intended to counter the renal sympathetic mediated sodium and water retention). These pharmacologic strategies, however, have significant limitations including limited efficacy, compliance issues, side effects, and others. Recently, intravascular devices that reduce sympathetic nerve activity by applying an energy field to a target site in the renal artery (e.g., via radiofrequency ablation) have been shown to reduce blood pressure in patients with treatment-resistant hypertension. WO 00/45706 relates to an intrabody HIFU applicator. WO 2012/120495 relates to an apparatus, which includes at least one ultrasound transducer that is configured to be positioned within a lumen of a subject and to ablate tissue surrounding a wall of the lumen without ablating the wall of the lumen, by focusing ultrasound energy on a focal zone that is outside of the wall of the lumen. WO 2007/134258 relates to a device for ablating body tissue.

### SUMMARY

The present technology is directed toward apparatus, systems and
exemplary methods for neuromodulation. Particularly, some embodiments are directed toward neuromodulation, including renal neuromodulation, through the application of ultrasonic energy at the treatment site. The ultrasonic energy can be applied by one or more transducers, such as, for example, polyvinylidene difluoride, or PVDF, transducers that are positioned proximate the artery wall and excited to emit ultrasonic energy. An energy source can be included to supply current to the ultrasonic transducer or transducers.

Various embodiments provide a catheter apparatus for treatment of a human patient via renal neuromodulation. The catheter apparatus can include a therapeutic assembly having a central axis and a distal portion and a proximal portion axially spaced along the central axis. The therapeutic assembly can include, for example, a catheter tip with one or more ultrasonic transducers diposed thereon and configured to emit ultrasonic energy in a direction toward the vessel wall at the treatment site. The catheter tip can be a flexible catheter tip and can be configured in a straight or biased (e.g., deflected) configuration. The tip can also be configured as an articulating or adjustable tip that can be reshaped or redirected in situ to allow positioning the transducer in a desired location and orientation relative to the vessel wall. The one or more transducers can be disposed on the catheter such that they are disposed on the surface of the catheter or disposed to be within or partially within the geometry of the catheter tip. The catheter and the transducers can be configured such that the transducers can be delivered to the treatment site and placed proximate the target tissue (e.g., vessel wall) for treatment. A transducer placed proximate a vessel wall can be positioned to come in to contact with the vessel wall in touching relation across all or part of a surface of the transducer. Alternatively a transducer can be positioned to be proximate, but not touching, the tissue.

Other features and aspects of the invention will become apparent from the following detailed description, taken in conjunction with the accompanying drawings, which illustrate, by way of example, the features in accordance with embodiments of the invention. The summary is not intended to limit the scope of the invention, which is defined solely by the claims attached hereto.

### BRIEF DESCRIPTION OF THE DRAWINGS

The present invention, in accordance with one or more various embodiments, is described in detail with reference to the accompanying figures. The drawings are provided for purposes of illustration only and merely depict typical or example embodiments of the invention. These drawings are provided to facilitate the reader's understanding of the systems and exemplary methods described herein, and shall not be considered limiting of the breadth, scope, or applicability of the claimed invention. The scope of the invention is defined by the independent claim. Preferred embodiments are given in the dependent claims.
FIG. 1 illustrates a system in accordance with one embodiment of the technology disclosed herein.
FIG. 2 illustrates one example of modulating renal nerves with an embodiment of the system described with reference to FIG. 1.
FIGS. 3A and 3B illustrate example configurations of a PVDF transducer in accordance with embodiments of the technology described herein.
FIGS. 4a and 4b are diagrams illustrating examples of electrically connecting signal leads to the PVDF transducer material in accordance with embodiments of the technology described herein.
FIG. 5a is a diagram illustrating a view of a PVDF transducer mounted in a flexible tip of a catheter in accordance with one embodiment of the technology described herein.
FIG. 5b is a diagram illustrating another view of the catheter shown in FIG. 5b.
FIG. 6 is a diagram illustrating a side view of a concave PVDF transducer mounted in a flexible tip of a catheter in accordance with one embodiment of the technology described herein.
FIGS. 7a, 7b and 7c are side view diagrams illustrating an example of a reconfigurable PVDF transducer in accordance with one embodiment of the technology described herein.
FIGS. 8a and 8b, are diagrams illustrating a PVDF transducer mounted in a deflected soft tip in accordance with one embodiment of the technology described herein.
FIGS. 9a and 9b are diagrams illustrating a PVDF transducer mounted in a tubular member in accordance with one embodiment of the technology described herein.
FIGS. 10 is a diagram illustrating an example of a via in a catheter tip in accordance with one embodiment of the technology described herein.
FIGS. 11a and 11b are diagrams illustrating example configurations of vias in a catheter tip in accordance with an embodiment of the technology described herein.
FIG. 12 is a diagram illustrating one example of a configuration using multiple transducers in accordance with one embodiment of the technology described herein.

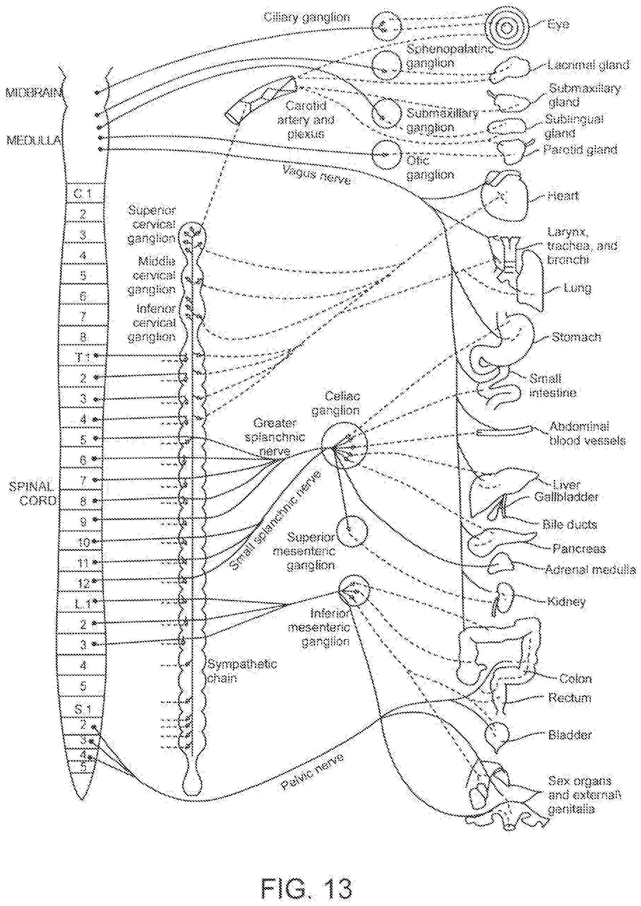

FIG. 13 illustrates a network of nerves that make up the sympathetic nervous system, allowing the brain to communicate with the body.
FIG. 14 illustrates the kidney, innervated by the renal plexus (RP), which is intimately associated with the renal artery.
FIGS. 15a and 15b, illustrate afferent communication from the kidney to the brain and from one kidney to the other kidney (via the central nervous system).
FIG. 16a shows human arterial vasculature.
FIG. 16b shows human venous vasculature.

The figures are not intended to be exhaustive or to limit the invention to the precise form disclosed. It should be understood that the invention can be practiced with modification and alteration, and that the invention be limited only by the claims and the equivalents thereof.

### DETAILED DESCRIPTION

The present technology is generally directed to modulation of nerves, including nerves innervating the kidneys. Various techniques can be used to partially or completely incapacitate neural pathways, such as those innervating the kidney. The purposeful application of energy to tissue can induce one or more desired thermal heating effects on localized regions of the renal artery and adjacent regions of the renal plexus (RP), which lay intimately within or adjacent to the adventitia of the renal artery. The purposeful application of the thermal heating and cooling effects can achieve neuromodulation along all or a portion of the renal plexus (RP). The technology disclosed herein is not limited to renal neuromodulation, but can be used for a number of neuromodulation targets. More generally, this technology can also be applied to tissue ablation for a variety of different ablation targets. Treatments could include any catheter based ablation procedure such as, for example, cardiac catheter ablation and tumor ablation. Cardiac catheter ablation could include the treatment of conditions such as, for example, atrial fibrillation, atrial flutter, ventricular tachycardia, supraventricular tachycardia (SVT) and Wolff-Parkinson-White syndrome.

Embodiments of the present technology are directed toward apparatus, systems and methods for neuromodulation. Particularly, some embodiments are directed toward neuromodulation, including renal neuromodulation, through the application of ultrasonic energy at the treatment site. The ultrasonic energy can be applied by one or more transducers, such as, for example, polyvinylidene difluoride, or PVDF, transducers that are positioned adjacent the artery wall and excited to emit ultrasonic energy. An energy source can be included to supply current to the ultrasonic transducer or transducers.

Various embodiments provide a catheter apparatus for treatment of a human patient via renal neuromodulation. The catheter apparatus can include a therapeutic assembly having a central axis and a distal portion and a proximal portion axially spaced along the central axis. The therapeutic assembly can include, for example, a catheter tip with one or more ultrasonic transducers diposed thereon and configured to emit ultrasonic energy in a direction toward the vessel wall at the treatment site. The catheter tip can be a flexible catheter tip and can be configured in a straight or biased (e.g., deflected) configuration. The tip can also be configured as an articulating or adjustable tip that can be reshaped or redirected in situ to allow positioning the transducer in a desired location and orientation relative to the vessel wall. The one or more ultrasonic transducers can be disposed on the catheter such that they are disposed on the surface of the catheter or disposed to be within or partially within the geometry of the catheter tip. The catheter and the transducers can be configured such that the transducers can be delivered to the treatment site and placed adjacent the target tissue (e.g., vessel wall) for treatment. A transducer placed adjacent a vessel wall can be positioned to come in to contact with the vessel wall in touching relation across all or part of a surface of the transducer. Alternatively a transducer can be positioned to be adjacent, but not touching, the tissue.

Specific details of several embodiments of the present technology are described herein with reference to the accompanying figures. Other embodiments of the present technology can have configurations, components, or procedures different from those described herein. For example, other embodiments can include additional elements and features beyond those described herein or be without several of the elements and features shown and described herein. Generally, unless the context indicates otherwise, the terms "distal" and "proximal" within this disclosure reference a position relative to an operator or an operator's control device. For example, "proximal" can refer to a position closer to an operator or an operator's control device, and "distal" can refer to a position that is more distant from an operator or an operator's control device. The headings provided herein are for convenience only.

### Renal Neuromodulation

Renal neuromodulation is the partial or complete incapacitation or other effective disruption of nerves innervating the kidneys. In particular, renal neuromodulation comprises inhibiting, reducing, and/or blocking neural communication along neural fibers (i.e., efferent and/or afferent nerve fibers) innervating the kidneys. Such incapacitation can be long-term (e.g., permanent or for periods of months, years, or decades) or short-term (e.g., for periods of minutes, hours, days, or weeks). Renal neuromodulation is expected to efficaciously treat several clinical conditions characterized by increased overall sympathetic activity, and in particular conditions associated with central sympathetic overstimulation such as hypertension, heart failure, acute myocardial infarction, metabolic syndrome, insulin resistance, diabetes, left ventricular hypertrophy, chronic and end stage renal disease, inappropriate fluid retention in heart failure, cardio-renal syndrome, osteoporosis and sudden death. The reduction of afferent neural signals contributes to the systemic reduction of sympathetic tone/drive, and renal neuromodulation is expected to be useful in treating several conditions associated with systemic sympathetic overactivity or hyperactivity. Renal neuromodulation can potentially benefit a variety of organs and bodily structures innervated by sympathetic nerves. For example, a reduction in central sympathetic drive may reduce insulin resistance that afflicts patients with metabolic syndrome and Type II diabetics.

### Selected Embodiments of Treatment Systems

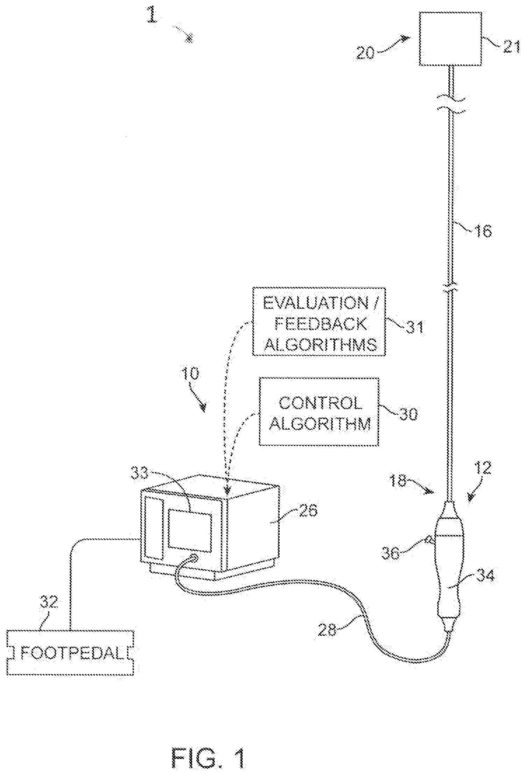

FIG. 1 illustrates a system 1 in accordance with an embodiment of the present technology. The system 1 includes a renal neuromodulation system 10 ("system 10"). The system 10 includes an intravascular or intraluminal treatment device 12 that is operably coupled to an energy source or console 26. Energy source or console 26 can include, for example, an RF signal generator or other energy source. In the embodiment shown in FIG.1, the treatment device 12 (e.g., a catheter) includes an elongated shaft 16 having a proximal portion 18, a handle 34 at a proximal region of the proximal portion 18, and a distal portion 20 extending distally relative to the proximal portion 18. The treatment device 12 further includes a therapeutic assembly or treatment section 21 at the distal portion 20 of the shaft 16. The therapeutic assembly 21 can include a neuromodulation assembly (e.g., actuators such as one or more PVDF transducers configured to deliver ultrasonic energy to the tissue.

Upon delivery to the target treatment site within the renal blood vessel, the therapeutic assembly 21 can be further configured to be placed into a treatment state or arrangement for delivering energy at the treatment site and providing therapeutically effective renal neuromodulation. In some embodiments, the therapeutic assembly 21 may be placed or transformed into the treatment state or arrangement via remote actuation, e.g., via an actuator 36, such as a knob, button, pin, or lever carried by the handle 34. In other embodiments, however, the therapeutic assembly 21 may be transformed between the delivery and treatment states using other suitable mechanisms or techniques.

The proximal end of the therapeutic assembly 21 is carried by or affixed to the distal portion 20 of the elongated shaft 16. A distal end of the therapeutic assembly 21 may terminate with, for example, an atraumatic rounded tip or cap. Alternatively, the distal end of the therapeutic assembly 21 may be configured to engage another element of the system 10 or treatment device 12. For example, the distal end of the therapeutic assembly 21 may define a passageway for engaging a guide wire (not shown) for delivery of the treatment device using over-the-wire ("OTW") or rapid exchange ("RX") techniques.

The energy source or console 26 is configured to generate a selected form and magnitude of energy (e.g., ultrasonic energy) for delivery to the target treatment site via the therapeutic assembly 21. A control mechanism, such as foot pedal 32 or other operator control, may be connected (e.g., pneumatically connected or electrically connected) to the console to allow the operator to initiate, terminate and, optionally, adjust various operational characteristics of the energy generator, including, but not limited to, power delivery.

The system 10 may also include a remote control device (not shown) that can be positioned in a sterile field and operably coupled to the therapeutic assembly 21. The remote control device can be configured to allow for selective activation of the therapeutic assembly 21. For example, the remote control device can be configured to allow the operator to initiate, terminate and, optionally, adjust various operational characteristics of the energy generator. In some embodiments, a control mechanism (not shown) may be built into the handle assembly 34 allowing operator control through actuation of buttons, switches or other mechanisms on the handle assembly 34.

The energy source 26 can be configured to deliver the treatment energy under the control of an automated control algorithm 30, under the control of the clinician, or via a combination thereof. In addition, the energy source or console 26 may include one or more evaluation or feedback algorithms 31 that can be configured to accept information and provide feedback to the clinician before, during, and/or after therapy (e.g., neuromodulation). Feedback can be provided in the form of audible, visual or haptic feedback. The feedback can be based on output from a monitoring system (not shown). The monitoring system can be a system including sensors or other monitoring devices integrated with treatment device 12, sensors or other monitoring devices separate from treatment device 12, or a combination thereof The monitoring devices of the monitoring system can be configured to measure conditions at the treatment site (e.g., the temperature of the tissue being treated), systemic conditions (e.g., patient vital signs), or other conditions germane to the treatment or to the health and safety of the patient.

The energy source 26 can further include a device or monitor that may include processing circuitry, such as one or more microprocessors, and a display 33. The processing circuitry may be configured to execute stored instructions relating to the control algorithm 30. The energy source 26 may be configured to communicate with the treatment device 12 (e.g., via the cable 28) to control the neuromodulation assembly and/or to send signals to or receive signals from the monitoring system. The display 33 may be configured to provide indications of power levels or sensor data, such as audio, visual or other indications, or may be configured to communicate the information to another device. For example, the console 26 may also be operably coupled to a catheter lab screen or system for displaying treatment information (e.g., nerve activity before and after treatment, effects of ablation, efficacy of ablation of nerve tissue, lesion location, lesion size, etc.).

The energy source or console 26 can be configured to control, monitor, supply, or otherwise support operation of the treatment device 12. In other embodiments, the treatment device 12 can be self-contained and/or otherwise configured for operation without connection to the energy source or console 26. As shown in the example of FIG. 1, the energy source or console 26 can include a primary housing having the display 33.

In some embodiments, the energy source or console 26 can include a processing device or module (not shown) having processing circuitry, e.g., a microprocessor. The processing device can be configured to execute stored instructions relating to the control algorithm 30, the evaluation/feedback algorithm 31 and other functions of the device. Furthermore, the energy source or console 26 can be configured to communicate with the treatment device 12, e.g., via the cable 28. For example, the therapeutic assembly 21 of the treatment device 12 can include a sensor (not shown) (e.g., a recording electrode, a temperature sensor, a pressure sensor, or a flow rate sensor) and a sensor lead (not shown) (e.g., an electrical lead or a pressure lead) configured to carry a signal from the sensor to the handle 34. The cable 28 can be configured to carry the signal from the handle 34 to the energy source or console 26.

The energy source or console 26 can have different configurations depending on the treatment modality of the treatment device 12. In embodiments described herein using an ultrasonic transducer, energy source or console 26 can include an RF energy generator used to excite the transducer(s).

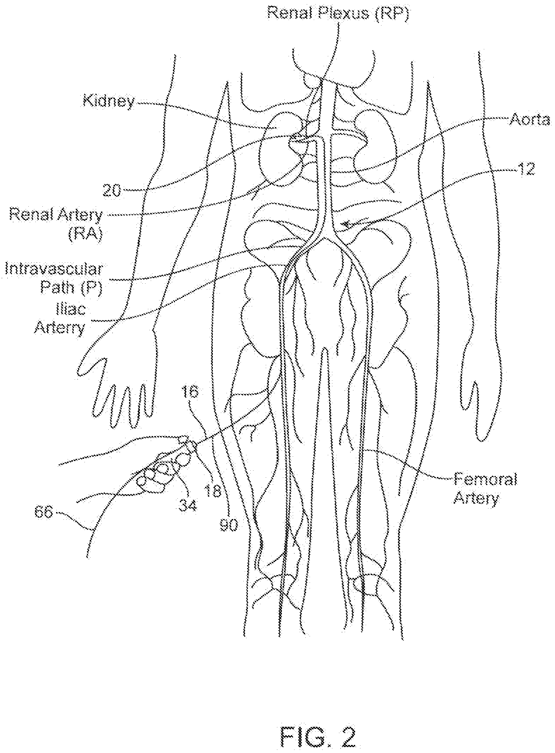

FIG. 2 illustrates one example of modulating renal nerves with an embodiment of the system 10. In this embodiment, the treatment device 12 provides access to the renal plexus (RP) through an intravascular path (P), such as a percutaneous access site in the femoral (illustrated), brachial, radial, or axillary artery to a targeted treatment site within a respective renal artery (RA). As illustrated, a section of the proximal portion 18 of the shaft 16 is exposed externally of the patient. By manipulating the proximal portion 18 of the shaft 16 from outside the intravascular path (P), the clinician may advance the shaft 16 through the sometimes tortuous intravascular path (P) and remotely manipulate the distal portion 20 of the shaft 16. Image guidance, e.g., computed tomography (CT), fluoroscopy, intravascular ultrasound (IVUS), optical coherence tomography (OCT), or another suitable guidance modality, or combinations thereof, may be used to aid the clinician's manipulation. Further, in some embodiments, image guidance components (e.g., IVUS, OCT) may be incorporated into the treatment device 12 itself.

After the therapeutic assembly 21 is adequately positioned in the renal artery (RA), it can be transitioned to the treatment state by the handle 34 or other suitable means until the neuromodulation assembly is positioned at its target site and the PVDF transducer is in a treatment position proximate the inner wall of the renal artery (RA). The purposeful application of energy (e.g., ultrasonic energy) from the neuromodulation assembly is then applied to tissue to induce one or more desired neuromodulating effects on localized regions of the renal artery and adjacent regions of the renal plexus (RP), which lay intimately within, adjacent to, or in close proximity to the adventitia of the renal artery (RA). The purposeful application of the energy may achieve neuromodulation along all or at least a portion of the renal plexus (RP).

In various embodiments, therapeutic assembly 21 can include one or ultrasonic transducers configured to direct ultrasonic energy at the treatment location to accomplish the desired neuromodulation effect. Particularly, in some embodiments, the application of ultrasonic energy from therapeutic assembly 21 can be made to the tissue to accomplish the desired treatment. Even more particularly, in such embodiments, ultrasonic energy can be applied to localized regions of the renal artery and adjacent regions of the renal plexus (RP), which lay intimately within, adjacent to, or in close proximity to the adventitia of the renal artery (RA) by the positioning of a ultrasonic transducer adjacent the arterial wall and causing the transducer to emit ultrasonic energy at a desired frequency and energy level.

One or more ultrasonic transducers can be included with and deployed by therapeutic assembly 21 in a number of different configurations. In various embodiments, ultrasonic transducers can be PVDF transducers configured to emit ultrasonic energy in response to energy generated by energy generator console 26 (FIG. 1). The ultrasonic transducers can be disposed on the catheter such that such that they are disposed on the surface of the catheter or disposed to be within or partially within the geometry of the catheter tip. The catheter and the transducers can be configured such that the transducers can be delivered to the treatment site and placed adjacent the target tissue (e.g., vessel wall) for treatment. A transducer placed adjacent a vessel wall can be positioned to come in to contact with the vessel wall in touching relation across all or part of a surface of the transducer. Alternatively a transducer can be positioned to be adjacent, but not touching, the tissue.

The delivery structure can be an expandable or deflectable structure that is used to position the ultrasonic transducer or transducers into position for deployment at the vessel wall. The delivery structure can include a catheter tip that can be configured to allow positioning of the ultrasonic transducers for treatment. The catheter tip can be a flexible catheter tip and can be configured in a straight or biased (e.g., deflected) configuration. The tip can also be configured as an articulating or adjustable tip that can be reshaped or redirected in situ to allow positioning the transducer in a desired location and orientation relative to the vessel wall.

As stated above, in some embodiments the transducer used for the ultrasound catheter can be a PVDF transducer. Because the renal nerves generally lie approximately 2-3mm from the surface of the arterial wall (e.g., beyond the endothelial wall), the PVDF transducer can be configured to transmit ultrasonic energy to shallow depths without excessively heating the transducer. For example, the PVDF transducer can be configured to transmit ultrasonic energy to depths in the range of 1-4mm. As another example, the PVDF transducer can be configured to transmit ultrasonic energy to depths in the range of 2-3mm. As yet another example, VDF transducer can be configured to transmit ultrasonic energy to depths in the range of 0.5-6mm.

PVDF is a non-reactive thermoplastic fluoropolymer produced by the polymerization of vinylidene difluoride. It is a ferroelectric polymer with a low acoustic impedance, similar to that of the body, making it suitable for renal neuromodulation applications. Its flexible mechanical properties mean that it can be shaped in a number of different geometries suitable for placement in a catheter or otherwise as part of therapeutic assembly 21. The PVDF material can be formed into a thin sheet of desired length, width and thickness. It is easy it is to manipulate in comparison to PZT, providing for cost effective manufacture, assembly and shaping. In some embodiments, either or both major surfaces of the sheet can be metallized. For example, in some embodiments a surface of the PVDF transducer is metallized with a thin CrAu metallic layer to which lead wires can be soldered or attached.

In addition, another advantage that can be gained by using PVDF is that the ablation profile can be made shallow relative to other energy modalities for ablation. This is because that PVDF generally performs better as a receiver than a transmitter (i.e., its efficiency coefficient for converting mechanical energy to electrical energy is better than its ability to convert electrical energy to mechanical energy). This provides an inherent safety mechanism to help avoid ablating deeper than intended and affecting non-target tissue. Accordingly, this technology is also well suited to ablation procedures that require shallower depths such as, for example ablation in an organ where nerves are closer to the organ inner cavity surface.

One other advantage of a PVDF transducer is that it operates with lower losses than PZT transducers and will generate less heat. This helps to keep the intima safe, and allows operation without additional cooling beyond that provided by bloodflow. Also, because PVDF couples better with the tissue/blood as compared to PZT, a higher percentage of its energy is coupled into the tissue.

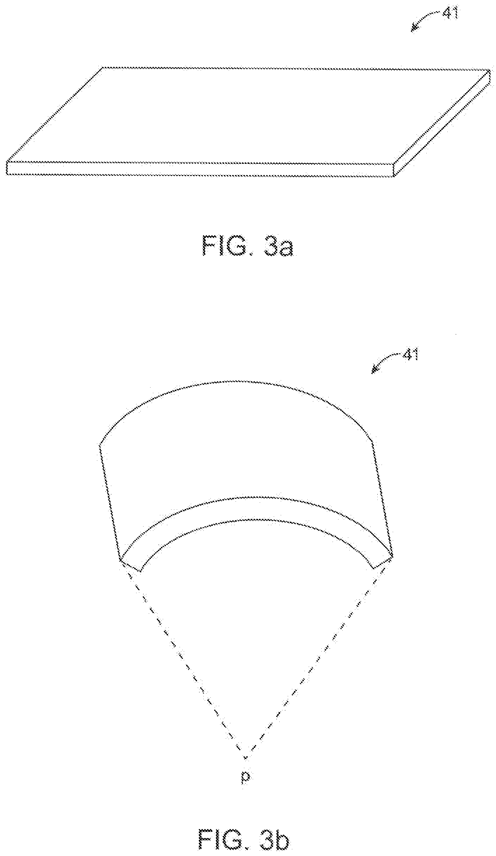

FIGS. 3A and 3B illustrate example PVDF transducer configurations in accordance with embodiments of the technology described herein. FIG. 3A illustrates a PVDF transducer 41 in a flat or substantially flat configuration. FIG. 3B illustrates a PVDF transducer 41 in a curved configuration. A curved configuration can be configured to form a concave energy source to focus the ultrasound energy to a focal point p. Because the nerves to be ablated generally lie approximately 2-3 mm from the surface of the artery wall, it may be desirable to use a concave transducer to focus the ultrasonic radiation to a focal point p designed to overlap the region. The thickness of the PVDF material determines its frequency of resonance, which determines the natural focus of the element (in an unshaped configuration) and the extent of the far field. Focusing the element, either through a lens or by shaping the transducer, changes the natural focus of the transducer. The final focus is a function of both the natural focus and on the curvature introduced by the focusing element.

That is, configurations can be provided such that when the catheter is positioned for treatment, the energy is focused at a point that is approximately 2-3 mm beyond the surface of the artery wall.

Because PVDF polymers can be made thin and tend to have good mechanical flexibility, other configurations in addition to flat and concave can be provided. For example, although not illustrated, a cylindrical transducer can be provided. A cylindrical transducer can be configured to radiate energy outward in all directions in a 360° pattern. It is noted that a cylindrical ablation pattern may not be desired as that may lead to stenosis formation. In further embodiments, the PVDF transducer can be formed in a helical geometry about the catheter so as to emit ultrasonic energy in a helical pattern.

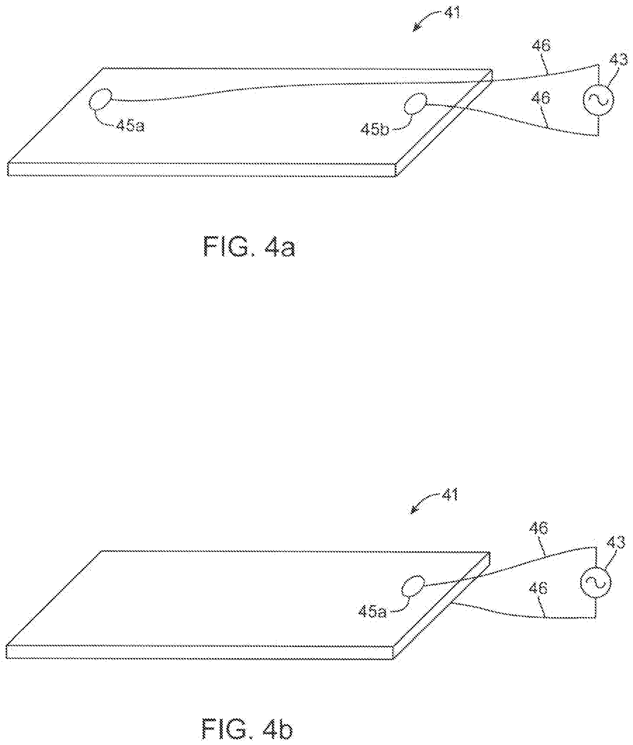

RF energy is delivered from an energy generator (e.g., located at energy source or console 26) to the PVDF transducer. For example, the RF signal can be delivered by signal leads from energy source or console 26 to the therapeutic assembly 21 via cable 28. The signal leads are electrically connected to the PVDF transducer such that the RF signal can be coupled to the PVDF material. FIGS. 4a and 4b are diagrams illustrating examples of electrically connecting signal leads to the PVDF transducer material.

Referring now to FIG. 4a, this example shows a pair of electrical contact pads 45a, 45b disposed on the surface of PVDF transducer 41. Electrical contact pads 45a, 45b can be metallic pads, allowing signal leads 46 to be soldered or otherwise electrically connected thereto. Electrical contact pads 45a, 45b can be disposed anywhere on PVDF transducer 41. In the examples illustrated in FIG. 4a, they are shown as being disposed on opposite ends of the top surface of PVDF transducer 41. In the embodiment illustrated in FIG. 4b, Electrical contact pads 45a, 45b are disposed on opposite surfaces of PVDF transducer 41. Contact pads can be printed 45a, 45b (e.g., screen printed) onto PVDF transducer 41 at the desired locations. Although not illustrated, one of ordinary skill in the art after reading this description will appreciate that similar electrode configurations can be applied to shaped transducers (e.g., to a curved transducer as shown in FIG. 3b).

Signal leads 46 are electrically connected to Electrical contact pads 45a, 45b at the distal end, and to signal generator 43 at the proximal end. Accordingly, signal leads can be run along cable 28 to couple the PVDF transducer 41 to energy source or console 26. Signal generator 43 can be a signal generated included with energy source or console 26. For example, energy source or console 26 can include a frequency synthesizer or DSP to generate an oscillating RF signal, and an amplifier to amplify the signal to desired levels for treatment. In embodiments where PVDF transducer is metallized, 41 electrical contact pads 45a, 45b are not needed and signal leads 46 can be connected directly to the metallized layer. However, in most applications, contact pads will be applied to allow a place at which signal leads 46 can be attached because the heat applied during the soldering process can damage the PVDF polymer material. Accordingly, electrical contact pads 45a, 45b will provide sufficient heat sink characteristics to prevent damage to the PVDF material by the heat of the soldering operation.

In various embodiments the PVDF transducer is mounted with therapeutic assembly 21 so that it can be positioned adjacent the vessel wall for treatment. In some embodiments, the transducer is mounted and positioning is made in such a way that the PVDF transducer does not contact the intima. This can allow blood to flow around or across the surface of the PVDF transducer. Such blood flow can carry heat away from the transducer, reducing the amount of heat that is conducted from the transducer to the intima. Further, the transducer is mounted to the catheter such that when positioned in the artery, the focal point of the transducer is at the desired depth in the targeted tissue. For example, where the targeted depth is 2-3mm based on the anticipated location of the renal nerves, the transducer is mounted on the catheter such that its focal point (or focal plane) is at a depth of approximately 2-3 mm when the catheter is positioned for treatment. Accordingly, in some embodiments, the transducer is mounted and the catheter is designed such that the transducer can be positioned at a distance from the vessel intima to allow the focal point or plane to be at the desired depth. In some embodiments, therapeutic assembly 21 can be configured such that the PVDF transducer can be placed from about 1-5 mm from the intima. In further embodiments, therapeutic assembly 21 can be configured such that the PVDF transducer can be placed from about 1-2 mm from the intima.

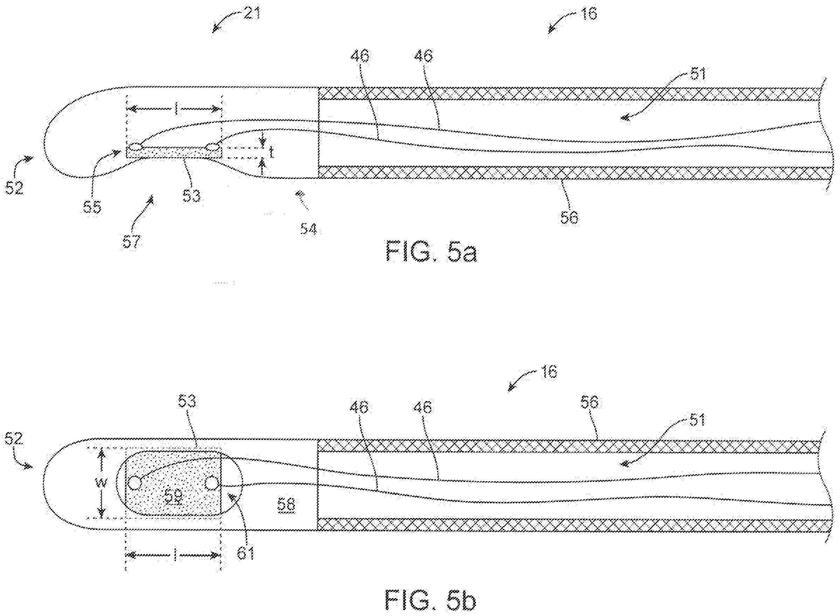

FIG. 5a is a diagram illustrating a side view of a PVDF transducer mounted in a flexible tip of a catheter in accordance with one embodiment of the technology described herein. Referring now to FIG. 5a, in this example a therapeutic assembly 21 is provided at the distal end of a catheter shaft 16. Catheter shaft 16 can be a braided polymer shaft 56 forming a lumen 51. Therapeutic assembly 21 comprises a flexible catheter tip 52 and a PVDF transducer 53 (e.g. PVDF transducer 41) mounted thereon. Flexible catheter tip 52 is configured to be positioned in the vessel such that the surface facing the bottom of the figure (the contact surface 58 shown in FIG. 5b) is placed in contact with the vessel wall.

In the example of FIG. 5a, PVDF transducer 53 is a sheetlike structure having a length 1, a width w (not shown) and a thickness t. PVDF transducer 53 has a distal end 55 and a proximal end 54. Electrodes (e.g., electrical contact pads 45a, 45b) are disposed on the surface of PVDF transducer 53 and a signal wire or lead 46 is connected to each electrode. Signal leads 46 can be routed from PVDF transducer 53, through lumen 51 to energy source or console 26.

In the example illustrated in FIG. 5a, the PVDF transducer 53 is mounted in a recess area 57 of the catheter tip of therapeutic assembly 21. As shown, the recess area 57 is a concave section of the catheter tip. This concavity can be provided to ensure (or reduce the chance) that PVDF transducer 53 does not contact the vessel wall. In embodiments not claimed, the recess area is not provided and the PVDF transducer 53 may be allowed to come into contact with the vessel wall. The geometry of the recess area 57 and the mounting of PVDF transducer 53 can be configured such that the PVDF transducer 53 is positioned at a desired distance from the intima when the contact surface 58 of catheter tip 52 is contacting the vessel wall. This can make the transducer easier to position a desired distance from the vessel wall. In other words, the depth of the concavity and the mounting of the PVDF transducer 53 are chosen such that the bottom (as oriented in the figure) surface of PVDF transducer 53 is a predetermined distance from the outer boundary of the contact surface 58. For example, in some embodiments the dimensions are chosen such that the PVDF transducer is from 1-5mm (and preferably 1-2mm or 1-3mm) from the intima when flexible catheter tip 52 is placed adjacent the vessel wall. The depth of the concavity in recess area 57 can be chosen based on the focal length of the PVDF transducer 53.

FIG. 5b is a diagram illustrating a top view of the PVDF transducer 53, and flexible tip 52, of the catheter shown in FIG. 5a. Particularly, FIG. 5b shows a view toward contact surface 58 of the catheter of FIG. 5a. As seen from this perspective, flexible catheter tip 52 includes an aperture 61 behind which PVDF transducer 53 is mounted. A portion of major surface 59 of PVDF transducer 53 is visible through aperture 61.

In various embodiments, PVDF transducer 53 can be mounted to a flexible catheter tip 52 using a number of techniques. These techniques can include, for example, using adhesives, securing PVDF transducer 53 with mechanical constraints, overmolding of the polymer, and the like. PVDF transducer 53 can be secured along all or part of its edges so as to not interfere with resonance of the material.

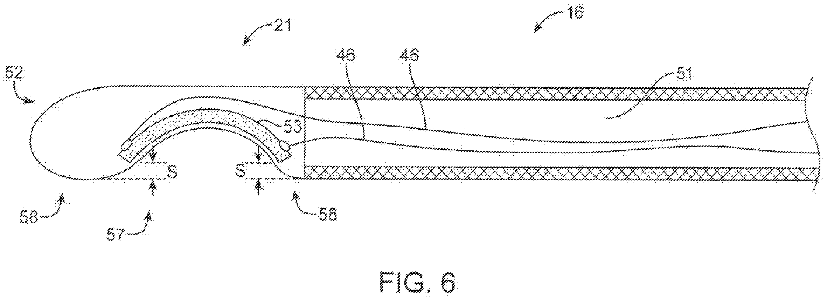

FIG. 6 is a diagram illustrating a side view of a concave PVDF transducer mounted in a flexible tip of a catheter in accordance with one embodiment of the technology described herein. In this example, PVDF transducer 53 is configured in a curved geometry. This provides a concave surface on the face of the PVDF transducer 53. The PVDF transducer 53 can be mounted such that its concave surface is faced toward the treatment site when the catheter tip is in position for treatment. PVDF transducer 53 can be configured such that its radius matches the radius of recess area 57 of flexible catheter tip 52.

PVDF transducer 53 can be mounted on flexible catheter tip 52 such that a spacing s is provided between the edges of PVDF transducer 53 and the contacting surface 58 of flexible catheter tip 52. This can be done to reduce the likelihood that PVDF transducer 53 will contact the intima during treatment. This can also provide an avenue for blood flow across the face of PVDF transducer 53, thereby reducing the amount of heat conducted to the vessel wall during treatment.

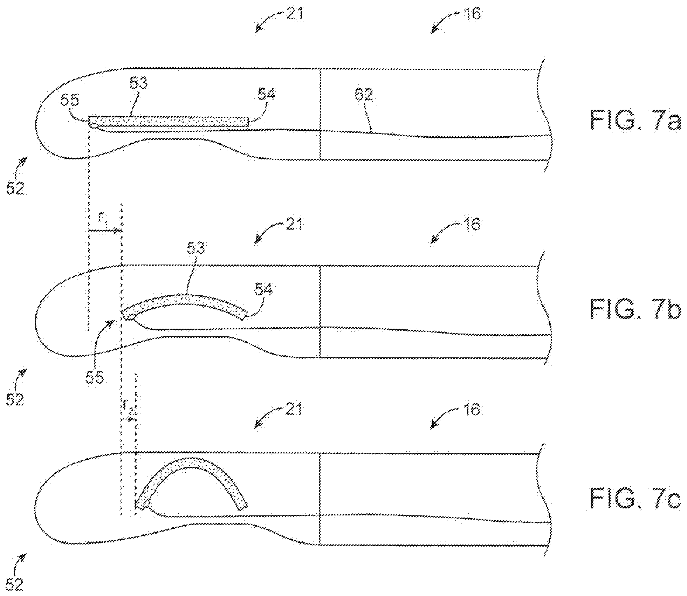

Because PVDF transducer 53 can be manufactured with mechanical flexibility, it can be reconfigured in situ. FIGS. 7a, 7b and 7c are side view diagrams illustrating an example of a reconfigurable PVDF transducer in accordance with one embodiment of the technology described herein. These examples illustrate a PVDF transducer 53 mounted in a flexible catheter tip 52 similar to that of FIGS. 5a and 5b. However, in these examples, a pull wire 62 is included to draw the proximal and distal ends 54, 55 of PVDF transducer 53 together to radius PVDF transducer 53 by a desired amount. Pull wire 62 can be a stainless steel or polymer thread controlled by a control mechanism (e.g., actuator 36 on handle 34) at the distal end of catheter assembly 12.

As shown in FIG. 7a, pull wire 62 is attached at or near distal end 55 of PVDF transducer 53. Pull wire 62 is in a first position such that PVDF transducer 53 is in a relatively flat configuration. There is little or no tension on pull wire 62, and PVDF transducer 53 is allowed to lie flat.

In FIG. 7b, pull wire 62 is retracted by an amount r1. Assuming little or no elasticity in pull wire 62, this draws distal end 55 of PVDF transducer 53 toward proximal end 54 of PVDF transducer 53 by amount r1. This causes arching of PVDF transducer 53, creating a concave surface. In FIG. 7c, pull wire 62 is retracted farther. This further draws distal end 55 of PVDF transducer 53 toward proximal end 54 of PVDF transducer 53 by amount r2. This creates a smaller radius and a larger degree of curvature (and concavity) in PVDF transducer 53. Accordingly, configurations such as this can be used to change the radius and hence the focal length of PVDF transducer 53. Such changes can be made before the start of the procedure, or when the therapeutic assembly 21 is positioned at the treatment site.

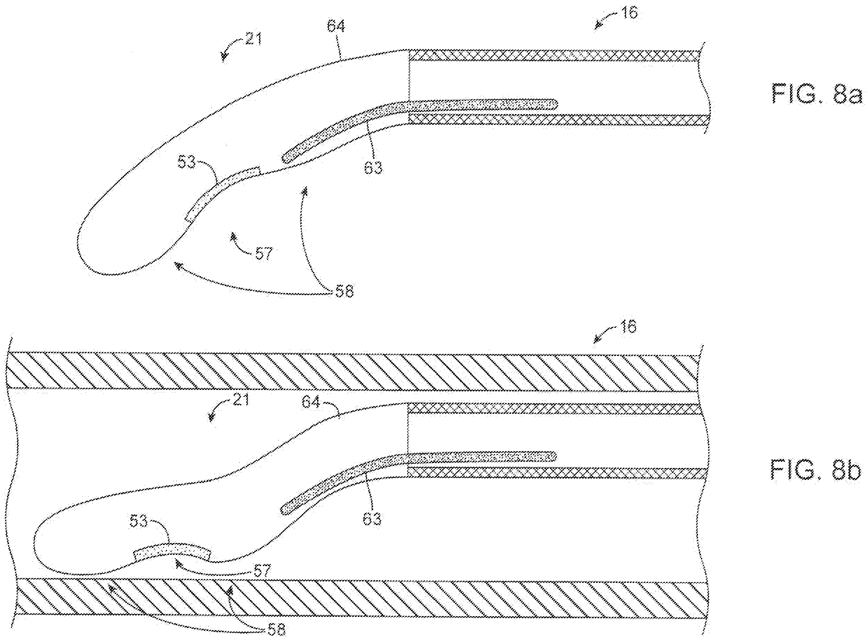

There are other configurations possible to allow placement of the PVDF transducer at or near the vessel wall. For example, as shown in FIGS. 8a and 8b, a PVDF transducer 53 can be mounted in a deflected or movable polymer soft tip 64. A flat or curved PVDF transducer 53 can be mounted in soft catheter tip 64 as described above with reference to FIGS. 5a, 5b, 6 and 7. Referring to FIG. 8a, the illustrated example includes an elongated delivery shaft 16 and a soft catheter tip 64 with a mandrel 63 positioned therein.

Mandrel 63 can be a shape set or preshaped mandrel 63 used to provide a predetermined curvature or deflection to soft catheter tip 62. For example, as shown in FIG. 8a, mandrel 63 is shaped so as to deflect soft catheter tip 64. Particularly, mandrel 63 can be shaped to cause a predetermined deflection of soft catheter tip 64 such that its contact surface 58 is deflected off axis, or angled away from catheter shaft 16. This can facilitate placement of the transducer adjacent a vessel wall. Mandrel 63 can be made from materials such as, for example spring steel or nitinol. In addition, a pull wire (not shown) or other mechanism can be used to deflect soft catheter tip 64 into position after delivery. This can allow the catheter to be in an axial, low profile configuration for delivery, and to be reconfigured into the deflected configuration upon positioning at the treatment site.

Referring now to FIG. 8b, shown is an example of a catheter with a polymer soft tip 64 positioned in a vessel (e.g., renal artery RA) for treatment. As this example illustrates, when inserted into renal artery RA, the soft catheter tip 64 partially straightens against the arterial wall. This places the PVDF transducer 53 into position for treatment. As shown in the illustrated example, contact surface 58 of soft catheter tip 64 is placed into contact with the intima. While in position, PVDF transducer 53 is spaced apart from and in a non-touching relation with the tissue. Additionally, blood is free to flow through recess area 57 between PVDF transducer 53 and the intima.

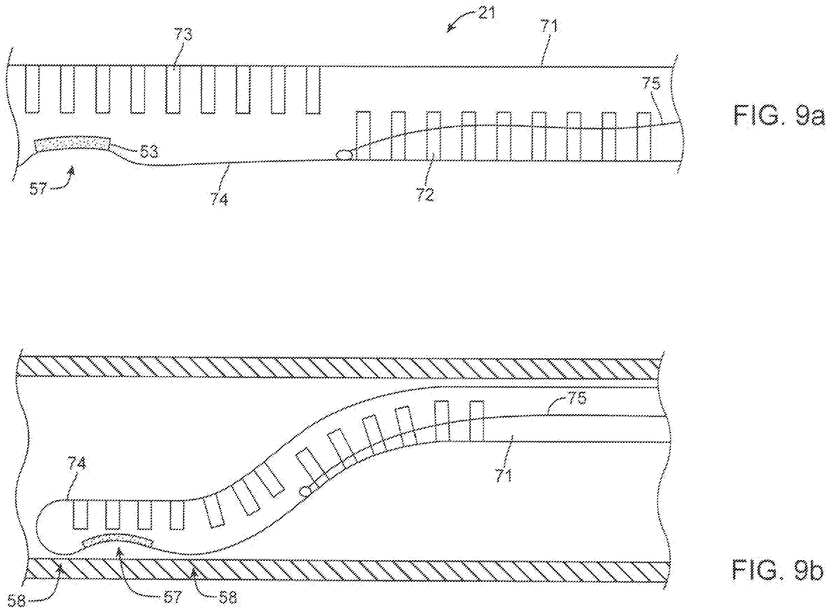

Above examples describe a PVDF transducer mounted in a soft catheter tip, such as a polymer catheter tip. As would be apparent to one of ordinary skill in the art after reading this description, other mounting structures can be used to mount one or more PVDF transducers. For example, one or more PVDF transducers can be mounted in a tubular member, such as hypotubing. FIGS. 9a and 9b are diagrams illustrating a PVDF transducer mounted in a tubular member in accordance with one embodiment of the technology described herein. Referring now to FIG. 9a, a therapeutic assembly 21 comprises a laser-cut tubular member in two sections. These are a proximal section 71 and a distal section 74. Proximal and distal sections 71, 74 can be made from a continuous section of tubing or from separate sections joined together. A PVDF transducer 53 can be mounted, as shown, in a recess area 57 of the tubing. In various embodiments, the tubular member can be a hypotube member and can be made from stainless steel (e.g., low-carbon stainless steel) or other non-bioreactive materials.

Proximal section 71 includes a plurality of openings 72 (only one labeled for clarity) on one side of the tubing. Distal section 74 also includes a plurality of openings 73 (only one labeled for clarity) on one side of the tubing. Openings 72, 73 can comprise a plurality of laser cut grooves or openings cut into the tubing. Openings 72, 73 are sections of removed material or gaps in the tubing and can extend from one edge of the tubing to approximately the midpoint of the tubing. Openings 72, 73 can be used to allow the tubing to be flexed (e.g., bent or radiused) in a given direction to a curved configuration. Although illustrated as slots cut to approximately the midpoint of the tubing in FIG. 9a, openings 72, 73 can be configured in any of a number of different geometries to impart flexibility to the tubing. For example, the openings 72, 73 could extend past the midpoint of the tubing and indeed, almost circumferentially about the tubing), and they could be narrow or wider and their pitch could be larger or smaller than the illustrated examples. Additionally, they can be non-uniform and they can take other shapes such as, for example, they could be helical, V-shaped, U-shaped, and so on. As these examples serve to illustrate, openings 72, 73 could take on any of a number of different configurations.

In the illustrated example, openings 72 are on the opposite side of the tubing from openings 73. This allows proximal section 71 to flex in a direction different from that of distal section 74. Also shown in the example of FIG. 9a is a pull wire 75. Pull wire 75 can be included and attached at or near the distal end of proximal section 71. In this configuration, pull wire 75 can be used to apply tension to proximal section 7 1 in a direction parallel to its axis. Pulling pull wire 75 in the proximal direction draws the distal end of proximal section 71 toward its proximal end, causing proximal section 71 to deflect. An example of this is described below with reference to FIG. 9b.

FIG. 9b is a diagram illustrating a deflected tubing in accordance with one embodiment of the technology described herein. As shown in FIG. 9b, proximal section 71 of the shaft is deflected downward (relative to the orientation of the drawing), directing the distal section 74 toward the vessel wall. This deflection is caused by tensioning pull wire 75, and is permitted by the presence of openings 72. Distal section 74 of the shaft deflects in the opposite direction. This deflection is caused by pressuring contact surface 58 against the vessel wall and is permitted by openings 73. Deflection of distal section 74 places the contact surface 58 in contact with the vessel wall of renal artery RA. This places PVDF transducer 53 in a treatment position relative to the vessel wall. Additionally, blood is free to flow through recess area 57 between PVDF transducer 53 and the intima. As with some other examples described herein, in the treatment position, PVDF transducer 53 can be placed in a spaced-apart, non-touching relation with the tissue. In some embodiments, the tubular member can be configured such that the PVDF transducer can be placed from about 1-5 mm from the intima. In further embodiments, therapeutic assembly 21 can be configured such that the PVDF transducer can be placed from about 1-2 mm from the intima.

Where the tubular member is made from stainless steel or a variety of 'memory' materials, when tension is released from pull wire 75, the hypotubing can return to its aligned configuration as shown in FIG. 9a. In some embodiments, pressure is applied through pull wire 75 to help return the hypotubing to its aligned state. Accordingly, the catheter can be maintained in a low profile configuration for delivery and removal, and flexed to a deployed state for treatment. In various embodiments, the openings 72, 73 are cut through the tubing such that blood can flow through the lumen and cool PVDF transducer 53.

In various embodiments, one or more vias or passages can be provided in the catheter tip to direct blood flow across or toward the PVDF transducer. Such vias or passages can be provided in any of a number of different configurations. Providing blood flow can help to cool the PVDF transducer during operation. For stainless steel or other like embodiments, the vias can be laser cut passages cut into the tubing. For a polymer tip, the vias can be introduced, for example, during the molding process.

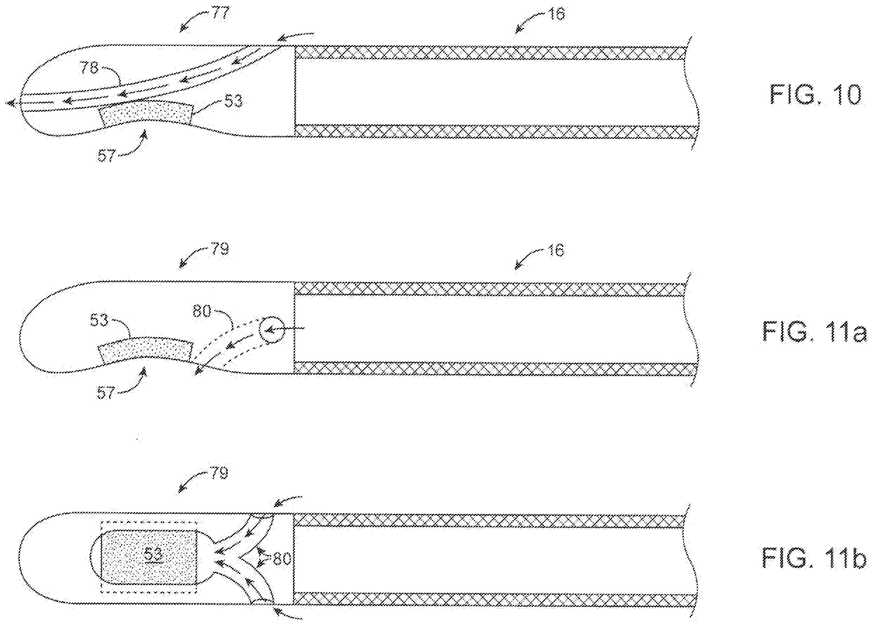

FIGS. 10, 11a and 11b are diagrams illustrating example configurations of vias in a catheter tip in accordance with various embodiments of the technology described herein. FIG. 10 illustrates a cutaway side view of a catheter tip 77 with a via 78. Via 78 is positioned within catheter tip 77 such that blood flows (indicated by arrows) through via 78 and across part or all of back surface of PVDF transducer 53. This allows the blood to carry heat away from PVDF transducer 53, lowering its operating temperature. As with other embodiments, blood can also flow through recess area 57, providing additional cooling of PVDF transducer 53.

FIGS. 11a and 11b are similar to FIG. 10a, but show an example of vias configured to take blood flow from the sides of the catheter tip. FIG. 11b shows a side view of a catheter tip 79 with a via 80 positioned to carry blood from the side of the catheter, over the front surface of PVDF transducer 53. The top view illustrated in FIG. 11b shows that via 80 can be a dual via configuration, carrying allowing blood to flow from both sides of catheter tip 79 to transducer 53. The embodiment shown in FIG. 10a can be combined with that shown in FIGS. 11a and 11b, providing vias to deliver blood flow to both major surfaces of PVDF transducer 53.

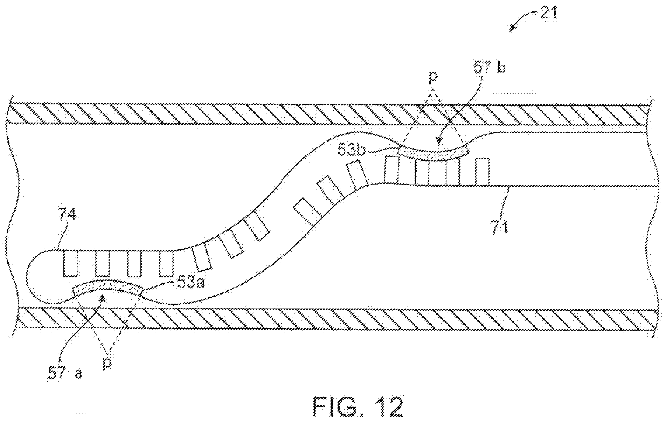

In various embodiments, multiple PVDF transducers can be provided with therapeutic assembly to allow simultaneous or sequential treatment at multiple treatment sites. FIG. 12 is a diagram illustrating one example of a configuration using multiple transducers in accordance with one embodiment of the technology described herein. In the example illustrated in FIG. 12, two PVDF transducers 53 are shown, distal transducer 53a and proximal transducer 53b. Although two are shown, a greater number of transducers can be provided. PVDF transducers 53a, 53b in this example are each shown disposed in their respective recess area 57a, 57b of therapeutic assembly 21. PVDF transducers 53a, 53b can be configured to direct the emitted energy in directions 180° apart from one another. PVDF transducers 53a, 53b can be powered at the same time or at different times. Additionally, they can each be powered at a respective duty cycle with overlapping or non-overlapping on times.

### Pertinent Anatomy and Physiology

The following discussion provides further details regarding pertinent patient anatomy and physiology. This section is intended to supplement and expand upon the previous discussion regarding the relevant anatomy and physiology, and to provide additional context regarding the disclosed technology and the therapeutic benefits associated with renal denervation. For example, as mentioned previously, several properties of the renal vasculature may inform the design of treatment devices and associated methods for achieving renal neuromodulation via intravascular access, and impose specific design requirements for such devices. Specific design requirements may include accessing the renal artery, facilitating stable contact between the energy delivery elements of such devices and a luminal surface or wall of the renal artery, and/or effectively modulating the renal nerves with the neuromodulatory apparatus.

### A. The Sympathetic Nervous System

The Sympathetic Nervous System (SNS) is a branch of the autonomic nervous system along with the enteric nervous system and parasympathetic nervous system. It is always active at a basal level (called sympathetic tone) and becomes more active during times of stress. Like other parts of the nervous system, the sympathetic nervous system operates through a series of interconnected neurons. Sympathetic neurons are frequently considered part of the peripheral nervous system (PNS), although many lie within the central nervous system (CNS). Sympathetic neurons of the spinal cord (which is part of the CNS) communicate with peripheral sympathetic neurons via a series of sympathetic ganglia. Within the ganglia, spinal cord sympathetic neurons join peripheral sympathetic neurons through synapses. Spinal cord sympathetic neurons are therefore called presynaptic (or preganglionic) neurons, while peripheral sympathetic neurons are called postsynaptic (or postganglionic) neurons.

At synapses within the sympathetic ganglia, preganglionic sympathetic neurons release acetylcholine, a chemical messenger that binds and activates nicotinic acetylcholine receptors on postganglionic neurons. In response to this stimulus, postganglionic neurons principally release noradrenaline (norepinephrine). Prolonged activation may elicit the release of adrenaline from the adrenal medulla.

Once released, norepinephrine and epinephrine bind adrenergic receptors on peripheral tissues. Binding to adrenergic receptors causes a neuronal and hormonal response. The physiologic manifestations include pupil dilation, increased heart rate, occasional vomiting, and increased blood pressure. Increased sweating is also seen due to binding of cholinergic receptors of the sweat glands.

The sympathetic nervous system is responsible for up- and down-regulating many homeostatic mechanisms in living organisms. Fibers from the SNS innervate tissues in almost every organ system, providing at least some regulatory function to things as diverse as pupil diameter, gut motility, and urinary output. This response is also known as sympatho-adrenal response of the body, as the preganglionic sympathetic fibers that end in the adrenal medulla (but also all other sympathetic fibers) secrete acetylcholine, which activates the secretion of adrenaline (epinephrine) and to a lesser extent noradrenaline (norepinephrine). Therefore, this response that acts primarily on the cardiovascular system is mediated directly via impulses transmitted through the sympathetic nervous system and indirectly via catecholamines secreted from the adrenal medulla.

Science typically looks at the SNS as an automatic regulation system, that is, one that operates without the intervention of conscious thought. Some evolutionary theorists suggest that the sympathetic nervous system operated in early organisms to maintain survival as the sympathetic nervous system is responsible for priming the body for action. One example of this priming is in the moments before waking, in which sympathetic outflow spontaneously increases in preparation for action.

### 1. The Sympathetic Chain

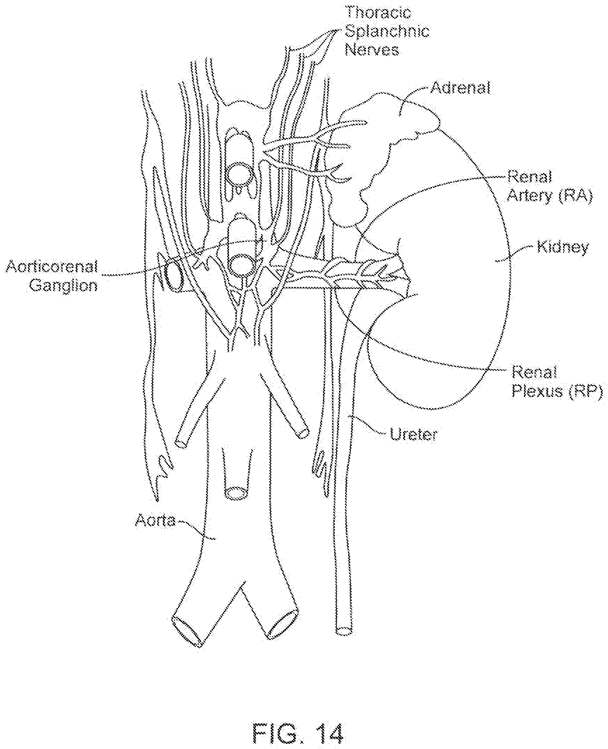

As shown in FIG. 14, the SNS provides a network of nerves that allows the brain to communicate with the body. Sympathetic nerves originate inside the vertebral column, toward the middle of the spinal cord in the intermediolateral cell column (or lateral horn), beginning at the first thoracic segment of the spinal cord and are thought to extend to the second or third lumbar segments. Because its cells begin in the thoracic and lumbar regions of the spinal cord, the SNS is said to have a thoracolumbar outflow. Axons of these nerves leave the spinal cord through the anterior rootlet/root. They pass near the spinal (sensory) ganglion, where they enter the anterior rami of the spinal nerves. However, unlike somatic innervation, they quickly separate out through white rami connectors which connect to either the paravertebral (which lie near the vertebral column) or prevertebral (which lie near the aortic bifurcation) ganglia extending alongside the spinal column.

In order to reach the target organs and glands, the axons should travel long distances in the body, and, to accomplish this, many axons relay their message to a second cell through synaptic transmission. The ends of the axons link across a space, the synapse, to the dendrites of the second cell. The first cell (the presynaptic cell) sends a neurotransmitter across the synaptic cleft where it activates the second cell (the postsynaptic cell). The message is then carried to the final destination.

In the SNS and other components of the peripheral nervous system, these synapses are made at sites called ganglia. The cell that sends its fiber is called a preganglionic cell, while the cell whose fiber leaves the ganglion is called a postganglionic cell. As mentioned previously, the preganglionic cells of the SNS are located between the first thoracic (T1) segment and third lumbar (L3) segments of the spinal cord. Postganglionic cells have their cell bodies in the ganglia and send their axons to target organs or glands.

The ganglia include not just the sympathetic trunks but also the cervical ganglia (superior, middle and inferior), which sends sympathetic nerve fibers to the head and thorax organs, and the celiac and mesenteric ganglia (which send sympathetic fibers to the gut).

### 2. Innervation of the Kidneys

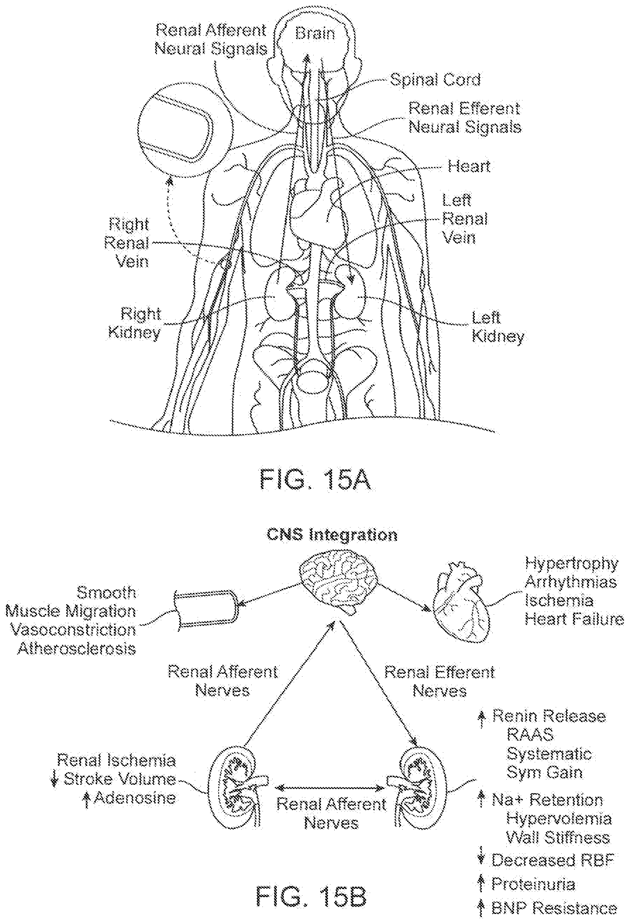

As shown in FIG. 15, the kidney is innervated by the renal plexus (RP), which is intimately associated with the renal artery. The renal plexus (RP) is an autonomic plexus that surrounds the renal artery and is embedded within the adventitia of the renal artery. The renal plexus (RP) extends along the renal artery until it arrives at the substance of the kidney. Fibers contributing to the renal plexus (RP) arise from the celiac ganglion, the superior mesenteric ganglion, the aorticorenal ganglion and the aortic plexus. The renal plexus (RP), also referred to as the renal nerve, is predominantly comprised of sympathetic components. There is no (or at least very minimal) parasympathetic innervation of the kidney.

Preganglionic neuronal cell bodies are located in the intermediolateral cell column of the spinal cord. Preganglionic axons pass through the paravertebral ganglia (they do not synapse) to become the lesser splanchnic nerve, the least splanchnic nerve, first lumbar splanchnic nerve, second lumbar splanchnic nerve, and travel to the celiac ganglion, the superior mesenteric ganglion, and the aorticorenal ganglion. Postganglionic neuronal cell bodies exit the celiac ganglion, the superior mesenteric ganglion, and the aorticorenal ganglion to the renal plexus (RP) and are distributed to the renal vasculature.

### 3. Renal Sympathetic Neural Activity

Messages travel through the SNS in a bidirectional flow. Efferent messages may trigger changes in different parts of the body simultaneously. For example, the sympathetic nervous system may accelerate heart rate; widen bronchial passages; decrease motility (movement) of the large intestine; constrict blood vessels; increase peristalsis in the esophagus; cause pupil dilation, piloerection (goose bumps) and perspiration (sweating); and raise blood pressure. Afferent messages carry signals from various organs and sensory receptors in the body to other organs and, particularly, the brain.

Hypertension, heart failure and chronic kidney disease are a few of many disease states that result from chronic activation of the SNS, especially the renal sympathetic nervous system. Chronic activation of the SNS is a maladaptive response that drives the progression of these disease states. Pharmaceutical management of the renin-angiotensin-aldosterone system (RAAS) has been a longstanding, but somewhat ineffective, approach for reducing over-activity of the SNS.

As mentioned above, the renal sympathetic nervous system has been identified as a major contributor to the complex pathophysiology of hypertension, states of volume overload (such as heart failure), and progressive renal disease, both experimentally and in humans. Studies employing radiotracer dilution methodology to measure overflow of norepinephrine from the kidneys to plasma revealed increased renal norepinephrine (NE) spillover rates in patients with essential hypertension, particularly so in young hypertensive subjects, which in concert with increased NE spillover from the heart, is consistent with the hemodynamic profile typically seen in early hypertension and characterized by an increased heart rate, cardiac output, and renovascular resistance. It is now known that essential hypertension is commonly neurogenic, often accompanied by pronounced sympathetic nervous system overactivity.

Activation of cardiorenal sympathetic nerve activity is even more pronounced in heart failure, as demonstrated by an exaggerated increase of NE overflow from the heart and the kidneys to plasma in this patient group. In line with this notion is the recent demonstration of a strong negative predictive value of renal sympathetic activation on all-cause mortality and heart transplantation in patients with congestive heart failure, which is independent of overall sympathetic activity, glomerular filtration rate, and left ventricular ejection fraction. These findings support the notion that treatment regimens that are designed to reduce renal sympathetic stimulation have the potential to improve survival in patients with heart failure.

Both chronic and end stage renal disease are characterized by heightened sympathetic nervous activation. In patients with end stage renal disease, plasma levels of norepinephrine above the median have been demonstrated to be predictive for both all-cause death and death from cardiovascular disease. This is also true for patients suffering from diabetic or contrast nephropathy. There is compelling evidence suggesting that sensory afferent signals originating from the diseased kidneys are major contributors to initiating and sustaining elevated central sympathetic outflow in this patient group; this facilitates the occurrence of the well known adverse consequences of chronic sympathetic over activity, such as hypertension, left ventricular hypertrophy, ventricular arrhythmias, sudden cardiac death, insulin resistance, diabetes, and metabolic syndrome.

### (i) Renal Sympathetic Efferent Activity

Sympathetic nerves to the kidneys terminate in the blood vessels, the juxtaglomerular apparatus and the renal tubules. Stimulation of the renal sympathetic nerves causes increased renin release, increased sodium (Na⁺) reabsorption, and a reduction of renal blood flow. These components of the neural regulation of renal function are considerably stimulated in disease states characterized by heightened sympathetic tone and clearly contribute to the rise in blood pressure in hypertensive patients. The reduction of renal blood flow and glomerular filtration rate as a result of renal sympathetic efferent stimulation is likely a cornerstone of the loss of renal function in cardio-renal syndrome, which is renal dysfunction as a progressive complication of chronic heart failure, with a clinical course that typically fluctuates with the patient's clinical status and treatment. Pharmacologic strategies to thwart the consequences of renal efferent sympathetic stimulation include centrally acting sympatholytic drugs, beta blockers (intended to reduce renin release), angiotensin converting enzyme inhibitors and receptor blockers (intended to block the action of angiotensin II and aldosterone activation consequent to renin release) and diuretics (intended to counter the renal sympathetic mediated sodium and water retention). However, the current pharmacologic strategies have significant limitations including limited efficacy, compliance issues, side effects and others.

### (ii) Renal Sensory Afferent Nerve Activity

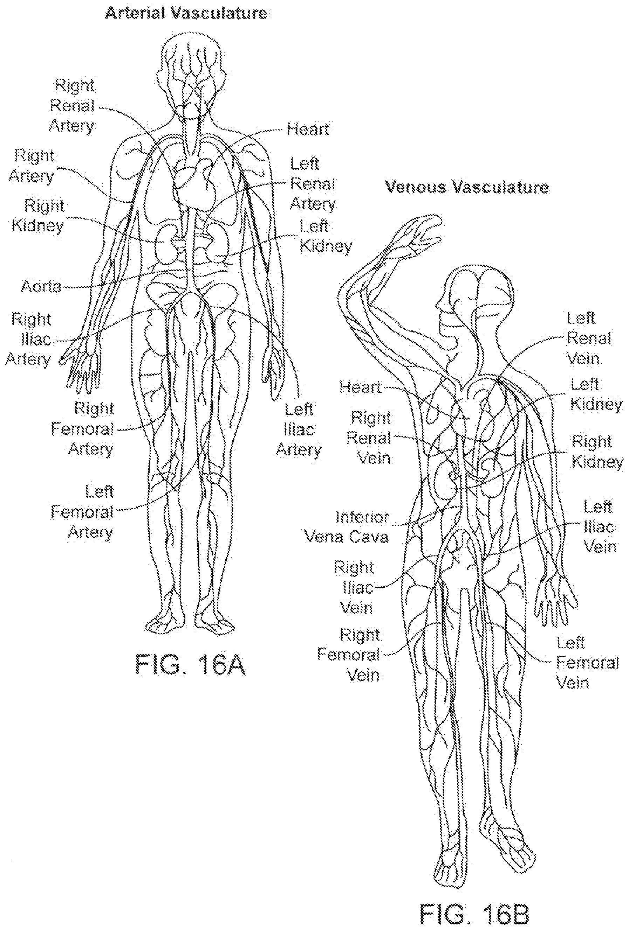

The kidneys communicate with integral structures in the central nervous system via renal sensory afferent nerves. Several forms of "renal injury" may induce activation of sensory afferent signals. For example, renal ischemia, reduction in stroke volume or renal blood flow, or an abundance of adenosine enzyme may trigger activation of afferent neural communication. As shown in FIGS. 16a and 16b, this afferent communication might be from the kidney to the brain or might be from one kidney to the other kidney (via the central nervous system). These afferent signals are centrally integrated and may result in increased sympathetic outflow. This sympathetic drive is directed towards the kidneys, thereby activating the RAAS and inducing increased renin secretion, sodium retention, volume retention and vasoconstriction. Central sympathetic over activity also impacts other organs and bodily structures innervated by sympathetic nerves such as the heart and the peripheral vasculature, resulting in the described adverse effects of sympathetic activation, several aspects of which also contribute to the rise in blood pressure.

The physiology therefore suggests that (i) modulation of tissue with efferent sympathetic nerves will reduce inappropriate renin release, salt retention, and reduction of renal blood flow, and that (ii) modulation of tissue with afferent sensory nerves will reduce the systemic contribution to hypertension and other disease states associated with increased central sympathetic tone through its direct effect on the posterior hypothalamus as well as the contralateral kidney. In addition to the central hypotensive effects of afferent renal denervation, a desirable reduction of central sympathetic outflow to various other sympathetically innervated organs such as the heart and the vasculature is anticipated.

### B. Additional Clinical Benefits of Renal Denervation

As provided above, renal denervation is likely to be valuable in the treatment of several clinical conditions characterized by increased overall and particularly renal sympathetic activity such as hypertension, metabolic syndrome, insulin resistance, diabetes, left ventricular hypertrophy, chronic end stage renal disease, inappropriate fluid retention in heart failure, cardio-renal syndrome, and sudden death. Since the reduction of afferent neural signals contributes to the systemic reduction of sympathetic tone/drive, renal denervation might also be useful in treating other conditions associated with systemic sympathetic hyperactivity. Accordingly, renal denervation may also benefit other organs and bodily structures innervated by sympathetic nerves. For example, as previously discussed, a reduction in central sympathetic drive may reduce the insulin resistance that afflicts people with metabolic syndrome and Type II diabetics. Additionally, patients with osteoporosis are also sympathetically activated and might also benefit from the down regulation of sympathetic drive that accompanies renal denervation.

### C. Achieving Intravascular Access to the Renal Artery

In accordance with the present technology, neuromodulation of a left and/or right renal plexus (RP), which is intimately associated with a left and/or right renal artery, may be achieved through intravascular access. As FIG. 17a shows, blood moved by contractions of the heart is conveyed from the left ventricle of the heart by the aorta. The aorta descends through the thorax and branches into the left and right renal arteries. Below the renal arteries, the aorta bifurcates at the left and right iliac arteries. The left and right iliac arteries descend, respectively, through the left and right legs and join the left and right femoral arteries.

As FIG. 17b shows, the blood collects in veins and returns to the heart, through the femoral veins into the iliac veins and into the inferior vena cava. The inferior vena cava branches into the left and right renal veins. Above the renal veins, the inferior vena cava ascends to convey blood into the right atrium of the heart. From the right atrium, the blood is pumped through the right ventricle into the lungs, where it is oxygenated. From the lungs, the oxygenated blood is conveyed into the left atrium. From the left atrium, the oxygenated blood is conveyed by the left ventricle back to the aorta.

As will be described in greater detail later, the femoral artery may be accessed and cannulated at the base of the femoral triangle just inferior to the midpoint of the inguinal ligament. A catheter may be inserted percutaneously into the femoral artery through this access site, passed through the iliac artery and aorta, and placed into either the left or right renal artery. This comprises an intravascular path that offers minimally invasive access to a respective renal artery and/or other renal blood vessels.

The wrist, upper arm, and shoulder region provide other locations for introduction of catheters into the arterial system. For example, catheterization of either the radial, brachial, or axillary artery may be utilized in select cases. Catheters introduced via these access points may be passed through the subclavian artery on the left side (or via the subclavian and brachiocephalic arteries on the right side), through the aortic arch, down the descending aorta and into the renal arteries using standard angiographic technique.

### D. Properties and Characteristics of the Renal Vasculature

Since neuromodulation of a left and/or right renal plexus (RP) may be achieved in accordance with the present technology through intravascular access, properties and characteristics of the renal vasculature may impose constraints upon and/or inform the design of apparatus, systems, and methods for achieving such renal neuromodulation. Some of these properties and characteristics may vary across the patient population and/or within a specific patient across time, as well as in response to disease states, such as hypertension, chronic kidney disease, vascular disease, end-stage renal disease, insulin resistance, diabetes, metabolic syndrome, etc. These properties and characteristics, as explained herein, may have bearing on the efficacy of the procedure and the specific design of the intravascular device. Properties of interest may include, for example, material/mechanical, spatial, fluid dynamic/hemodynamic and/or thermodynamic properties.

As discussed previously, a catheter may be advanced percutaneously into either the left or right renal artery via a minimally invasive intravascular path. However, minimally invasive renal arterial access may be challenging, for example, because as compared to some other arteries that are routinely accessed using catheters, the renal arteries are often extremely tortuous, may be of relatively small diameter, and/or may be of relatively short length. Furthermore, renal arterial atherosclerosis is common in many patients, particularly those with cardiovascular disease. Renal arterial anatomy also may vary significantly from patient to patient, which further complicates minimally invasive access. Significant inter-patient variation may be seen, for example, in relative tortuosity, diameter, length, and/or atherosclerotic plaque burden, as well as in the take-off angle at which a renal artery branches from the aorta. Apparatus, systems and methods for achieving renal neuromodulation via intravascular access should account for these and other aspects of renal arterial anatomy and its variation across the patient population when minimally invasively accessing a renal artery.

In addition to complicating renal arterial access, specifics of the renal anatomy also complicate establishment of stable contact between neuromodulatory apparatus and a luminal surface or wall of a renal artery. When the neuromodulatory apparatus includes an energy delivery element, such as an electrode, consistent positioning and appropriate contact force applied by the energy delivery element to the vessel wall are important for predictability. However, navigation is impeded by the tight space within a renal artery, as well as tortuosity of the artery. Furthermore, establishing consistent contact is complicated by patient movement, respiration, and/or the cardiac cycle because these factors may cause significant movement of the renal artery relative to the aorta, and the cardiac cycle may transiently distend the renal artery (i.e., cause the wall of the artery to pulse).

Even after accessing a renal artery and facilitating stable contact between neuromodulatory apparatus and a luminal surface of the artery, nerves in and around the adventia of the artery should be safely modulated via the neuromodulatory apparatus. Effectively applying thermal treatment from within a renal artery is non-trivial given the potential clinical complications associated with such treatment. For example, the intima and media of the renal artery are highly vulnerable to thermal injury. As discussed in greater detail below, the intima-media thickness separating the vessel lumen from its adventitia means that target renal nerves may be multiple millimeters distant from the luminal surface of the artery. Sufficient energy should be delivered to or heat removed from the target renal nerves to modulate the target renal nerves without excessively cooling or heating the vessel wall to the extent that the wall is frozen, desiccated, or otherwise potentially affected to an undesirable extent. A potential clinical complication associated with excessive heating is thrombus formation from coagulating blood flowing through the artery. Given that this thrombus may cause a kidney infarct, thereby causing irreversible damage to the kidney, thermal treatment from within the renal artery should be applied carefully. Accordingly, the complex fluid mechanics and thermodynamic conditions present in the renal artery during treatment, particularly those that may impact heat transfer dynamics at the treatment site, may be important in applying energy (e.g., heating thermal energy) and/or removing heat from the tissue (e.g., cooling thermal conditions) from within the renal artery.

The neuromodulatory apparatus should also be configured to allow for adjustable positioning and repositioning of the energy delivery element within the renal artery since location of treatment may also impact clinical efficacy. For example, it may be tempting to apply a full circumferential treatment from within the renal artery given that the renal nerves may be spaced circumferentially around a renal artery. In some situations, full-circle lesion likely resulting from a continuous circumferential treatment may be potentially related to renal artery stenosis. Therefore, the formation of more complex lesions along a longitudinal dimension of the renal artery via the mesh structures described herein and/or repositioning of the neuromodulatory apparatus to multiple treatment locations may be desirable. It should be noted, however, that a benefit of creating a circumferential ablation may outweigh the potential of renal artery stenosis or the risk may be mitigated with certain embodiments or in certain patients and creating a circumferential ablation could be a goal. Additionally, variable positioning and repositioning of the neuromodulatory apparatus may prove to be useful in circumstances where the renal artery is particularly tortuous or where there are proximal branch vessels off the renal artery main vessel, making treatment in certain locations challenging. Manipulation of a device in a renal artery should also consider mechanical injury imposed by the device on the renal artery. Motion of a device in an artery, for example by inserting, manipulating, negotiating bends and so forth, may contribute to dissection, perforation, denuding intima, or disrupting the interior elastic lamina.

Blood flow through a renal artery may be temporarily occluded for a short time with minimal or no complications. However, occlusion for a significant amount of time should be avoided to prevent injury to the kidney such as ischemia. It could be beneficial to avoid occlusion all together or, if occlusion is beneficial to the embodiment, to limit the duration of occlusion, for example to 2-5 minutes.

Based on the above described challenges of (1) renal artery intervention, (2) consistent and stable placement of the treatment element against the vessel wall, (3) effective application of treatment across the vessel wall, (4) positioning and potentially repositioning the treatment apparatus to allow for multiple treatment locations, and (5) avoiding or limiting duration of blood flow occlusion, various independent and dependent properties of the renal vasculature that may be of interest include, for example, (a) vessel diameter, vessel length, intima-media thickness, coefficient of friction, and tortuosity; (b) distensibility, stiffness and modulus of elasticity of the vessel wall; (c) peak systolic, end-diastolic blood flow velocity, as well as the mean systolic-diastolic peak blood flow velocity, and mean/max volumetric blood flow rate; (d) specific heat capacity of blood and/or of the vessel wall, thermal conductivity of blood and/or of the vessel wall, and/or thermal convectivity of blood flow past a vessel wall treatment site and/or radiative heat transfer; (e) renal artery motion relative to the aorta induced by respiration, patient movement, and/or blood flow pulsatility; and (f) the take-off angle of a renal artery relative to the aorta. These properties will be discussed in greater detail with respect to the renal arteries. However, dependent on the apparatus, systems and methods utilized to achieve renal neuromodulation, such properties of the renal arteries, also may guide and/or constrain design characteristics.

As noted above, an apparatus positioned within a renal artery should conform to the geometry of the artery. Renal artery vessel diameter, DRA, typically is in a range of about 2-10 mm, with most of the patient population having a DRA of about 4mm to about 8mm and an average of about 6 mm. Renal artery vessel length, LRA, between its ostium at the aorta/renal artery juncture and its distal branchings, generally is in a range of about 5-70 mm, and a significant portion of the patient population is in a range of about 20-50mm. Since the target renal plexus is embedded within the adventitia of the renal artery, the composite Intima-Media Thickness, IMT, (i.e., the radial outward distance from the artery's luminal surface to the adventitia containing target neural structures) also is notable and generally is in a range of about 0.5-2.5 mm, with an average of about 1.5 mm. Although a certain depth of treatment is important to reach the target neural fibers, the treatment should not be too deep (e.g., > 5 mm from inner wall of the renal artery) to avoid non-target tissue and anatomical structures such as the renal vein.

An additional property of the renal artery that may be of interest is the degree of renal motion relative to the aorta, induced by respiration and/or blood flow pulsatility. A patient's kidney, which located at the distal end of the renal artery, may move as much as 4" (10.16 cm) cranially with respiratory excursion. This may impart significant motion to the renal artery connecting the aorta and the kidney, thereby requiring from the neuromodulatory apparatus a unique balance of stiffness and flexibility to maintain contact between the thermal treatment element and the vessel wall during cycles of respiration. Furthermore, the take-off angle between the renal artery and the aorta may vary significantly between patients, and also may vary dynamically within a patient, e.g., due to kidney motion. The take-off angle generally may be in a range of about 30 degrees-135 degrees.

### Conclusion

While various embodiments of the present invention have been described above, it should be understood that they have been presented by way of example only, and not of limitation. Likewise, the various diagrams may depict an example architectural or other configuration for the invention, which is done to aid in understanding the features and functionality that can be included in the invention. The invention is not restricted to the illustrated example architectures or configurations, but the desired features can be implemented using a variety of alternative architectures and configurations. Indeed, it will be apparent to one of skill in the art how alternative functional, logical or physical partitioning and configurations can be implemented to implement the desired features of the present invention. Also, a multitude of different constituent module names other than those depicted herein can be applied to the various partitions. Additionally, with regard to flow diagrams and operational descriptions, the order in which the steps are presented herein shall not mandate that various embodiments be implemented to perform the recited functionality in the same order unless the context dictates otherwise.

Although the invention is described above in terms of various exemplary embodiments and implementations, it should be understood that the various features, aspects and functionality described in one or more of the individual embodiments are not limited in their applicability to the particular embodiment with which they are described, but instead can be applied, alone or in various combinations, to one or more of the other embodiments of the invention, whether or not such embodiments are described and whether or not such features are presented as being a part of a described embodiment. Thus, the breadth and scope of the present invention should not be limited by any of the above-described exemplary embodiments but by the appended independent claim.

Terms and phrases used in this document, and variations thereof, unless otherwise expressly stated, should be construed as open ended as opposed to limiting. As examples of the foregoing: the term "including" should be read as meaning "including, without limitation" or the like; the term "example" is used to provide exemplary instances of the item in discussion, not an exhaustive or limiting list thereof; the terms "a" or "an" should be read as meaning "at least one," "one or more" or the like; and adjectives such as "conventional," "traditional," "normal," "standard," "known" and terms of similar meaning should not be construed as limiting the item described to a given time period or to an item available as of a given time, but instead should be read to encompass conventional, traditional, normal, or standard technologies that may be available or known now or at any time in the future. Likewise, where this document refers to technologies that would be apparent or known to one of ordinary skill in the art, such technologies encompass those apparent or known to the skilled artisan now or at any time in the future.

The presence of broadening words and phrases such as "one or more," "at least," "but not limited to" or other like phrases in some instances shall not be read to mean that the narrower case is intended or required in instances where such broadening phrases may be absent. The use of the term "module" does not imply that the components or functionality described or claimed as part of the module are all configured in a common package. Indeed, any or all of the various components of a module, whether control logic or other components, can be combined in a single package or separately maintained and can further be distributed in multiple groupings or packages or across multiple locations.

Additionally, the various embodiments set forth herein are described in terms of exemplary block diagrams, flow charts and other illustrations. As will become apparent to one of ordinary skill in the art after reading this document, the illustrated embodiments and their various alternatives can be implemented without confinement to the illustrated examples. For example, block diagrams and their accompanying description should not be construed as mandating a particular architecture or configuration.

## Claims

1. An ultrasound apparatus (12) for thermally-induced renal neuromodulation, the apparatus comprising:
a catheter (16) configured for intravascular placement within a renal blood vessel of a human patient; and
a therapeutic assembly (21) positioned along the catheter (16), wherein the therapeutic assembly (21) comprises a polyvinylidene difluoride (PVDF) ultrasound transducer (41, 53) configured to transmit ultrasound waves to target neural fibers along the renal blood vessel to achieve thermal renal neuromodulation via heating,
and a recessed portion (57),
wherein the PVDF ultrasound transducer is configured to transmit the ultrasound waves over less than a full 360° about the blood vessel,
wherein the therapeutic assembly is coupled to a flexible distal end portion (20) of the catheter (16), and wherein the PVDF ultrasound transducer is positioned within the recessed portion (57) of the therapeutic assembly (21), and wherein the therapeutic assembly (21) is configured to engage at least a portion of a wall of the renal blood vessel during transmission of the ultrasound waves to the target neural fibers, and wherein the recessed portion (57) of the therapeutic assembly (21) is sized and shaped such that the PVDF ultrasound transducer (41, 53) positioned within the recess remains out of contact with the wall of the renal blood vessel during treatment.

2. The ultrasound apparatus (12) of claim 1 wherein the PVDF ultrasound transducer (41, 53) configured to be positioned approximately 1-5 mm from the wall the renal blood vessel during therapy such that a focal plane of the PVDF ultrasound transducer (41, 53) has a desired depth beyond the wall of the renal blood vessel.

3. The ultrasound apparatus (12) of claim 1 wherein the focal plane of the PVDF ultrasound transducer (41, 53) is configured to be approximately 1-4 mm beyond the wall of the renal blood vessel.

4. The ultrasound apparatus (12) of claim 1 wherein the catheter (16) is sized and shaped such that it does not occlude blood flow in the renal blood vessel during therapy.

5. The ultrasound apparatus (12) of claim 1 wherein the therapeutic assembly comprises a first side carrying the PVDF ultrasound transducer (41, 53) and a second side opposite the first side, and wherein, during therapy, the therapeutic assembly is configured to be positioned within the renal blood vessel for therapy such that the first side faces a wall of the renal blood vessel without contacting the wall.

6. The ultrasound apparatus (12) of claim 1 wherein the PVDF ultrasound transducer (41, 53) comprises a flat or substantially flat plate carried by the therapeutic assembly (21).

7. The ultrasound apparatus (12) of claim 1 wherein the PVDF ultrasound transducer (41, 53) comprises a curved or shaped plate carried by the therapeutic assembly (21).

8. The ultrasound apparatus (12) of claim 1, wherein the catheter (16) has
an elongated shaft (16) having a proximal portion (18) and a distal portion (20); and wherein
the therapeutic assembly (21) is at the distal portion (20) of the elongated shaft (16) and is sized to be located within a renal artery (RA) of a human patient,
wherein the PVDF ultrasound transducer (41, 53) is configured to be shaped in situ relative to the wall of the renal artery (RA), and wherein the shaped PVDF ultrasound transducer (41, 53) is configured to transmit ultrasound energy to a localized region of target neural tissue adjacent the wall of the renal artery (RA).

9. The ultrasound apparatus (12) of claim 8 wherein the PVDF ultrasound transducer (41, 53) comprises a planar or generally planar structure, or wherein the PVDF ultrasound transducer (41, 53) comprises a curved structure having a generally concave arrangement.

10. The ultrasound apparatus (12) of claim 8 wherein the PVDF ultrasound transducer (41, 53) is configured to remain out of contact with the wall of the renal artery (RA) during treatment.

11. The ultrasound apparatus (12) of claim 8 wherein the PVDF ultrasound transducer (41, 53) comprises a first shape before or during therapy, and wherein the PVDF ultrasound transducer (41, 53) is configured to be modified from the first shape to a separate shape while the therapeutic assembly is located within the renal artery (RA) of the patient.

12. The ultrasound apparatus (12) of claim 8 wherein the distal portion (20) of the elongated shaft (16) is flexible and configured to be shaped in situ to modify the position of the PVDF ultrasound transducer (41, 53) relative to the wall of the renal artery (RA).

## Patentansprüche

1. Ultraschallgerät (12) zur thermisch induzierten renalen Neuromodulation, wobei das Gerät umfasst:
einen Katheter (16), ausgelegt für die intravaskuläre Platzierung in einem renalen Blutgefäß eines menschlichen Patienten; und
eine therapeutische Anordnung (21), die entlang des Katheters (16) positioniert ist, wobei die therapeutische Anordnung (21) einen Polyvinylidendifluorid (PVDF)-Ultraschallwandler (41, 53), der ausgelegt ist zum Übertragen von Ultraschallwellen, zum Zielen auf neuronale Fasern entlang des renalen Blutgefäßes, zum Erreichen einer thermischen renalen Neuromodulation durch Erwärmen umfasst, und einen vertieften Abschnitt (57),
wobei der PVDF-Ultraschallwandler ausgelegt ist, um die Ultraschallwellen über weniger als volle 360° um das Blutgefäß zu übertragen,
wobei die therapeutische Anordnung mit einem flexiblen distalen Endabschnitt (20) des Katheters (16) gekoppelt ist, und wobei der PVDF-Ultraschallwandler innerhalb des vertieften Abschnitts (57) der therapeutischen Anordnung (21) positioniert ist, und wobei die therapeutische Anordnung (21) ausgelegt ist, um mindestens einen Abschnitt einer Wand des renalen Blutgefäßes während der Übertragung der Ultraschallwellen auf die Zielnervenfasern zu erfassen, und wobei der vertiefte Abschnitt (57) der therapeutischen Anordnung (21) so bemessen und geformt ist, dass der innerhalb der Vertiefung positionierte PVDF-Ultraschallwandler (41, 53) während der Behandlung außerhalb des Kontakts mit der Wand des renalen Blutgefäßes bleibt.

2. Ultraschallgerät (12) nach Anspruch 1, wobei der PVDF-Ultraschallwandler (41, 53) ausgelegt ist, um während der Therapie etwa 1-5 mm von der Wand des renalen Blutgefäßes entfernt positioniert zu werden, sodass eine Fokalebene des PVDF-Ultraschallwandlers (41, 53) eine gewünschte Tiefe außerhalb der Wand des renalen Blutgefäßes aufweist.

3. Ultraschallgerät (12) nach Anspruch 1, wobei die Fokalebene des PVDF-Ultraschallwandlers (41, 53) so ausgelegt ist, dass sie etwa 1-4 mm über der Wand des renalen Blutgefäßes liegt.

4. Ultraschallgerät (12) nach Anspruch 1, wobei der Katheter (16) so bemessen und geformt ist, dass er während der Therapie den Blutfluss im renalen Blutgefäß nicht verschließt.

5. Ultraschallgerät (12) nach Anspruch 1, wobei die therapeutische Anordnung eine erste Seite, die den PVDF-Ultraschallwandler (41, 53) trägt, und eine zweite Seite gegenüber der ersten Seite umfasst, und wobei die therapeutische Anordnung während der Therapie ausgelegt ist, um innerhalb des renalen Blutgefäßes für die Therapie so positioniert zu werden, dass die erste Seite einer Wand des renalen Blutgefäßes zugewandt ist, ohne die Wand zu berühren.

6. Ultraschallgerät (12) nach Anspruch 1, wobei der PVDF-Ultraschallwandler (41, 53) eine flache oder im Allgemeinen flache Platte umfasst, die von der therapeutischen Anordnung (21) getragen wird.

7. Ultraschallgerät (12) nach Anspruch 1, wobei der PVDF-Ultraschallwandler (41, 53) eine gekrümmte oder geformte Platte umfasst, die von der therapeutischen Anordnung (21) getragen wird.

8. Ultraschallgerät (12) nach Anspruch 1, wobei der Katheter (16)
einen länglichen Schaft (16) mit einem proximalen Abschnitt (18) und einem distalen Abschnitt (20) hat; und wobei
die therapeutische Anordnung (21) sich am distalen Abschnitt (20) des länglichen Schaftes (16) befindet und so bemessen ist, dass sie sich in einer renalen Arterie (RA) eines menschlichen Patienten befindet, wobei der PVDF-Ultraschallwandler (41, 53) so ausgelegt ist, dass er in situ in Bezug auf die Wand der renalen Arterie (RA) geformt ist, und wobei der geformte PVDF-Ultraschallwandler (41, 53) so ausgelegt ist, dass er Ultraschallenergie auf einen lokalisierten Bereich des Zielnervengewebes benachbart zur Wand der renalen Arterie (RA) überträgt.

9. Ultraschallgerät (12) nach Anspruch 8, wobei der PVDF-Ultraschallwandler (41, 53) eine planare oder im Allgemeine planare Struktur umfasst, oder wobei der PVDF-Ultraschallwandler (41, 53) eine gekrümmte Struktur mit einer im Allgemeinen konkaven Anordnung umfasst.

10. Ultraschallgerät (12) nach Anspruch 8, wobei der PVDF-Ultraschallwandler (41, 53) ausgelegt ist, um während der Behandlung außerhalb des Kontakts mit der Wand der renalen Arterie (RA) zu bleiben.

11. Ultraschallgerät (12) nach Anspruch 8, wobei der PVDF-Ultraschallwandler (41, 53) eine erste Form vor oder während der Therapie umfasst, und wobei der PVDF-Ultraschallwandler (41, 53) ausgelegt ist, um von der ersten Form in eine separate Form geändert zu werden, während sich die therapeutische Anordnung in der renalen Arterie (RA) des Patienten befindet.

12. Ultraschallgerät (12) nach Anspruch 8, wobei der distale Abschnitt (20) des verlängerten Schaftes (16) flexibel und ausgelegt ist, um in situ geformt zu werden, um die Position des PVDF-Ultraschallwandlers (41, 53) relativ zur Wand der renalen Arterie (RA) zu modifizieren.

## Revendications

1. Appareil à ultrasons (12) pour la neuromodulation rénale thermiquement induite, l'appareil comprenant :
un cathéter (16) conçu pour la pose intravasculaire à l'intérieur d'un vaisseau sanguin rénal d'un patient humain ; et
un ensemble thérapeutique (21) positionné le long du cathéter (16), dans lequel l'ensemble thérapeutique (21) comprend un transducteur ultrasonique (41, 53) en difluorure de polyvinylidène (PVDF) conçu pour transmettre des ondes ultrasoniques pour cibler des fibres neurales le long du vaisseau sanguin rénal pour réaliser une neuromodulation rénale thermique par chauffage, et une partie évidée (57),
dans lequel le transducteur ultrasonique PVDF est conçu pour transmettre les ondes ultrasoniques sur moins qu'un tour complet de 360° autour du vaisseau sanguin,
dans lequel l'ensemble thérapeutique est relié à une partie terminale distale flexible (20) du cathéter (16), et dans lequel le transducteur ultrasonique PVDF est positionné à l'intérieur de la partie évidée (57) de l'ensemble thérapeutique (21), et dans lequel l'ensemble thérapeutique (21) est conçu pour entrer en prise avec au moins une partie d'une paroi du vaisseau sanguin rénal au cours de la transmission des ondes ultrasoniques aux fibres neurales cibles, et dans lequel la partie évidée (57) de l'ensemble thérapeutique (21) est dimensionnée et formée de sorte que le transducteur ultrasonique PVDF (41, 53) positionné à l'intérieur de l'évidement reste hors du contact avec la paroi du vaisseau sanguin rénal au cours du traitement.

2. Appareil à ultrasons (12) selon la revendication 1, dans lequel le transducteur ultrasonique PVDF (41, 53) est conçu pour être positionné à environ 1 à 5 mm de la paroi du vaisseau sanguin rénal au cours du traitement de sorte qu'un plan focal du transducteur ultrasonique PVDF (41, 53) possède une profondeur souhaitée au-delà de la paroi du vaisseau sanguin rénal.

3. Appareil à ultrasons (12) selon la revendication 1, dans lequel le plan focal du transducteur ultrasonique PVDF (41, 53) est conçu pour être à environ 1 à 4 mm au-delà de la paroi du vaisseau sanguin rénal.

4. Appareil à ultrasons (12) selon la revendication 1, dans lequel le cathéter (16) est dimensionné et formé de sorte qu'il n'obture pas le flux sanguin dans le vaisseau sanguin rénal au cours du traitement.

5. Appareil à ultrasons (12) selon la revendication 1, dans lequel l'ensemble thérapeutique comprend un premier côté supportant le transducteur ultrasonique PVDF (41, 53) et un second côté opposé au premier côté, et dans lequel, au cours du traitement l'ensemble thérapeutique est conçu pour être positionné à l'intérieur du vaisseau sanguin rénal pour le traitement de sorte que le premier côté fait face à une paroi du vaisseau sanguin rénal sans entrer en contact avec la paroi.

6. Appareil à ultrasons (12) selon la revendication 1, dans lequel le transducteur ultrasonique PVDF (41, 53) comprend une plaque plate ou sensiblement plate supportée par l'ensemble thérapeutique (21).

7. Appareil à ultrasons (12) selon la revendication 1, dans lequel le transducteur ultrasonique PVDF (41, 53) comprend une plaque incurvée ou formée supportée par l'ensemble thérapeutique (21).

8. Appareil à ultrasons (12) selon la revendication 1, dans lequel le cathéter (16) possède
un arbre allongé (16) ayant une partie proximale (18) et une partie distale (20) ; et dans lequel
l'ensemble thérapeutique (21) se trouve à une partie distale (20) de l'arbre allongé (16) et il est dimensionné pour être positionné à l'intérieur de l'artère rénale (AR) d'un patient humain,
dans lequel le transducteur ultrasonique PVDF (41, 53) est conçu pour être formé in situ par rapport à la paroi de l'artère rénale (AR), et dans lequel le transducteur ultrasonique PVDF (41, 53) formé est conçu pour transmettre de l'énergie ultrasonique à une région localisée du tissu neurale cible adjacente à la paroi de l'artère rénale (AR).

9. Appareil à ultrasons (12) selon la revendication 8, dans lequel le transducteur ultrasonique PVDF (41, 53) comprend une structure plane ou généralement plane, ou dans lequel le transducteur ultrasonique PVDF (41, 53) comprend une structure incurvée ayant un agencement généralement concave.

10. Appareil à ultrasons (12) selon la revendication 8, dans lequel le transducteur ultrasonique PVDF (41, 53) est conçu pour rester hors du contact de la paroi de l'artère rénale (AR) au cours du traitement.

11. Appareil à ultrasons (12) selon la revendication 8, dans lequel le transducteur ultrasonique PVDF (41, 53) comprend une première forme avant ou au cours du traitement, et dans lequel le transducteur ultrasonique PVDF (41, 53) est conçu pour être modifié de la première forme vers une forme distincte lorsque l'ensemble thérapeutique est situé à l'intérieur de l'artère rénale (AR) du patient.

12. Appareil à ultrasons (12) selon la revendication 8, dans lequel la partie distale (20) de l'arbre allongé (16) est flexible et conçue pour être formée in situ pour modifier la position du transducteur ultrasonique PVDF (41, 53) par rapport à la paroi de l'artère rénale (AR).
